# EUROPEAN PATENT APPLICATION

(11) **EP 2 407 478 A1**
(43) Date of publication of application: **18.01.2012**
(21) Application number: 10169580.7
(22) Date of filing: 14.07.2010
(51) Int. Cl.: C07K 5/10, C07K 5/12, A61P 17/02, A61P 9/00, A61P 35/00

(54) **New cyclotetrapeptides with pro-angiogenic properties**

(71) Applicant: GENETADI Biotech, S.L., 48160 Derio (ES)
(72) Inventor: Aizpurua Iparraguirre, Jesus Maria, 20009, Donostia (ES); Oyarbide Vicuna, Joseba, 20110, Pasai San Pedro (ES); Fernandez Oyon, Xabier, 20013, Donostia (ES); Miranda Murua, Jose Ignacio, 20012, Donostia (ES); Gamboa Landa, Jose Ignacio, 20730, Azpeitia (ES); Avila Flores, Silvia, 48993, Getxo (ES); Castrillo Diez, Jose Luis, 48993, Getxo (ES)
(74) Representative: ZBM Patents

(57) **Abstract**

New cyclotetrapeptides of formula (I) or their pharmaceutically acceptable salts,
cyclo [Arg-Asp-(beta-Lactam)]
(I)
wherein (beta-Lactam) is a biradical of the formula (II) 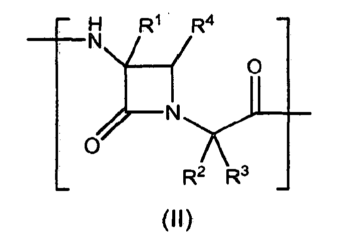 wherein the terminal NH group of the (beta-Lactam) is attached to the α-carbonyl group of the aspartic residue (Asp), and the terminal carbonyl group of the (beta-Lactam) is attached to the α-amino group the arginine residue (Arg); processes for their preparation, and pharmaceutical compositions containing them, as well as the said cyclotetrapeptides for use in human and animal therapy.

## Description

The present invention relates to new cyclotetrapeptides and their pharmaceutically acceptable salts, to processes for their preparation, and to the pharmaceutical compositions containing them, as well as to their use in human and animal therapy. It also relates to their use in the purification of integrins, or in the detection and/or quantification of integrins.

### BACKGROUND ART

Cell adhesion and interaction properties within different cell types and with the components of the extracellular matrix are essential for cell function. Integrins are αβ dimeric glycoprotein receptors of the cell wall playing an important role in such processes.

For instance, α_{V}β₃ integrin is an RGD-dependent receptor of endothelial cells preferentially expressed in angiogenic veins. It has been shown to play an important role during neovascularization and its antagonists block the formation of new blood vessels without affecting the pre-existing ones.

Vertebrates built on an entirely new set of adhesion-related genes involved in the development, maintenance, function, and regeneration of the vasculature, i.e. ECM proteins (for instance fibronectin, vitronectin, and fibrinogen) and their heterodimeric integrin adhesive receptors. Up to now, 9 of out of 24 vertebrate integrin heterodimers have been implicated in blood vessel formation, α_{V}β₃ integrin as major player in this vessel formation. Platelet aggregation, tumoral cell migration, metastasis, angiogenesis and osteoclast adhesion to the bone matrix depend on the interaction between integrin-type receptors and the proteins of the extracellular matrix mentioned above.

Several integrins recognize the hydrophilic amino acid sequence RGD Arg-Gly-Asp. Substrate-immobilized RGD sequences have been used to enhance cell binding to artificial surfaces. These molecules consist of an oligomeric structure formed by α helical coiled coil peptides fused at their amino-terminal ends with an RGD-containing fragment. When immobilized on a substrate, these peptides specifically promoted integrin α_{V}β₃-dependent cell adhesion. Synthetic peptides containing the RGD sequence might compete favorably with proteins in binding receptors (integrins). It has been shown that conformation of the RGD sequence is crucial for specific binding to the receptor.

Certain RGD cyclopentapeptide mimetics have been described as α_{V}β₃ integrin inhibitors, for example, the cyclo[Arg-Gly-Asp-(beta-Lactam)] described in WO 2006/048473, and the cyclo(Arg-Gly-Asp-(D)Phe-N(Me)Val) (also known as Cilengitide) disclosed in EP 770622.

Antiintegrin antibodies immobilized on a substrate have been used as agonist of integrin function. Contortrostatin, a snake venom disintegrin that is highly effective at stimulating signaling through several integrins, has also been used as agonist over α_{V}β₃ integrin.

Thus, antibodies, peptides or peptidomimetics activating selectively cell adhesion mechanisms controlled by one or several integrins and, more particularly by α_{V}β₃ integrin, are angiogenic agents.

Therefore, it still persists the need for active pro-angiogenic compounds.

### SUMMARY OF THE INVENTION

As mentioned above, cyclopentapeptides comprising the aminoacid sequence Arg-Gly-Asp (RGD) have been described in the prior art as integrin α_{V}β₃ inhibitors. These compounds show antiangiogenic properties.

The inventors have now surprisingly found that when the glycine (Gly) residue of these prior art cyclopentapeptides is not present, the resulting cyclotetrapeptides comprising the aminoacid sequence Arg-Asp (RD) not only show integrin adhesion properties comparable to that of the prior art compounds, but an unexpected opposite biological response. As it will be seen in more detail in the examples, the cyclotetrapeptides of the invention show pro-angiogenic properties.

Further, because of their size, the cyclotetrapeptides of the invention have the advantage that have a stable conformation and display good pharmacological properties.

Therefore, a first aspect of the present invention refers to compounds of formula (I), stereoisomers or mixtures thereof, or pharmaceutically acceptable salts thereof
cyclo[Arg-Asp-(beta-Lactam)] (I
wherein (beta-Lactam) is a biradical of the formula (II) wherein: 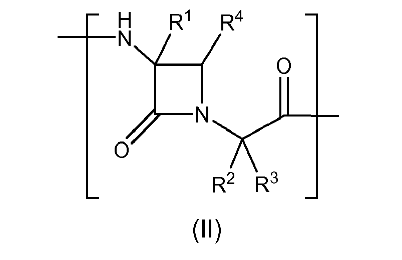
the terminal NH group of the (beta-Lactam) is attached to the α-carbonyl group of the aspartic residue (Asp), and the terminal carbonyl group of the (beta-Lactam) is attached to the α-amino group the arginine residue (Arg);
R¹, R², R³ and R⁴ are radicals independently selected from the group consisting of hydrogen, (C₁-C₁₂)alkyl, (C₂-C₁₂)alkenyl, (C₂-C₁₂)alkynyl, (C₃-C₁₀)cycloalkyl, (C₂-C₉)heterocycloalkyl, (C₆-C₁₂)aryl, and (C₅-C₁₁)heteroaryl; wherein the radicals alkyl, alkenyl and alkynyl are optionally substituted with one or more substituents selected from halogen, -CN, -OR^{a}, -SR^{a}, -SOR^{a}, -SO₂R^{a}-, -NO₂, -N₃, -NR^{a}R^{b}, -COR^{a}, -CONR^{a}R^{b}, (C₆-C₁₂)aryl, and (C₅-C₁₁)heteroaryl; and the radicals cycloalkyl, heterocycloalkyl, aryl, and heteroaryl are optionally substituted with one or more substituents selected from halogen, (C₁-C₄)alkyl optionally substituted with one or more halogen atoms, -CN, -OR^{a}, -SR^{a}, -SOR^{a}, -SO₂R^{a}-, -NO₂, -N₃, -NR^{a}R^{b}, -COR^{a} and -CONR^{a}R^{b}, and 2,3,4,6-tetra-O-benzyl-α-D-mannosyl;
or alternatively, R² and R³ together with the carbon atom to which they are attached form a 3- to 7-membered monocyclic carbocyclic ring, partially unsaturated or saturated, optionally containing one or more heteroatoms selected from the group consisting of N, O and S, and being optionally substituted with one or more substituents selected from halogen, (C₁-C₄)alkyl optionally substituted with one or more halogen atoms, -CN, -OR^{a}, -SR^{a}, -SOR^{a}, -SO₂R^{a}-, -NO₂, -N₃, -NR^{a}R^{b}, -COR^{a} and -CONR^{a}R^{b}; and
R^{a} and R^{b} are independently H or (C₁-C₁₂)alkyl.

Another aspect of the present invention relates to a process for the preparation of the compounds of formula (I) as defined above, which comprises:
a) reacting a compound of formula H-Z-OH (III) with a condensing agent, optionally in the presence of a base, wherein Z is a biradical selected from the group consisting of -Arg(R⁵)-Asp(R⁶)-(beta-Lactam)-, -Asp(R⁶)-(beta-Lactam)-Arg(R⁵)-, and -(beta-Lactam)-Arg(R⁵)-Asp(R⁶)-; R⁵ is H or an amino protecting group PG¹; R⁶ is H or a carboxyl protecting group PG²; and (beta-Lactam) is a biradical of the formula (II) as defined above; and
b) if necessary, removing the protecting groups PG¹ and PG²; and
c) optionally converting a basic or acidic compound of the formula (I) obtained in step a) or b) into one of its salts by treatment with an acid or base.

Another aspect of the invention relates to the intermediate compounds of formula (III) as defined above.

Another aspect of the invention relates to the intermediate compounds of formula (I')
cyclo[Arg(R⁵)-Asp(R⁶)-(beta-Lactam)] (I')
wherein R⁵, R⁶ and (beta-Lactam) are as previously defined.

Another aspect of the present invention relates to a pharmaceutical composition which comprises an effective amount of a compound of formula (I) as defined above, together with one or more pharmaceutically acceptable excipients or carriers.

As previously mentioned, the compounds of the invention show pro-angiogenic properties through interaction with the α_{V}β₃ integrin. Therefore, another aspect of the present invention relates to a compound of formula (I) as defined above for use in medicine.

In particular, the compounds of formula (I) as defined above may be used in the treatment of α_{V}β₃ integrin agonist mediated diseases. Therefore, another aspect of the present invention relates to a compound of formula (I) as defined above for use in the treatment of α_{V}β₃ integrin agonist mediated diseases. This aspect relates thus to the use of a compound of formula (I) as defined above for the manufacture of a medicament for use in the treatment of α_{V}β₃ integrin agonist mediated diseases.

It also forms part of the invention a method for treating a subject suffering from an α_{V}β₃ integrin agonist mediated disease, comprising administering a pharmaceutically effective amount of a compound of formula (I) as defined above, together with pharmaceutically acceptable excipients or carriers, in a subject in need thereof.

Another aspect of the present invention relates to a compound of formula (I) as defined above for use in the treatment of conditions which comprise the stimulation of the angiogenesis, such as bone formation around implants or vascular network traumas. This aspect relates thus to the use of a compound of formula (I) as defined above for the manufacture of a medicament for use in the treatment of conditions which comprise the stimulation of the angiogenesis, such as bone formation around implants or vascular network traumas.

It also forms part of the invention a method for treating a subject suffering from a condition which requires the stimulation of the angiogenesis, such as bone formation around implants or vascular network traumas, comprising administering a pharmaceutically effective amount of a compound of formula (I) as defined above, together with pharmaceutically acceptable excipients or carriers, in a subject in need thereof.

Another aspect of the present invention relates to a compound of formula (I) as defined above for use in the treatment of diabetic ulcers, blood vessels wounds on heart, or human melanomas. This aspect relates thus to the use of a compound of formula (I) as defined above for the manufacture of a medicament for use in the treatment of diabetic ulcers, blood vessels wounds on heart, or human melanomas.

It also forms part of the invention a method for treating a subject suffering from diabetic ulcers, blood vessels wounds on heart, or human melanomas, comprising administering a pharmaceutically effective amount of a compound of formula (I) as defined above, together with pharmaceutically acceptable excipients or carriers, in a subject in need thereof.

Another aspect of the invention relates to the use of the compounds of formula (I) as defined above as radiolabeled imaging agents for noninvasive imaging of integrin expression or as a thrombous imaging agent; as delivery agents of cytotoxins, liposomes, genes and fluorescent agents to tissues; or as ligands in the surface coating of materials in implant surgery as well as in cell culturing for tissue engineering; or as coatings for growth and physiological functioning of the cells on the biomaterial making up the tissue or organ that is to be replaced.

### BRIEF DESCRIPTION OF THE DRAWINGS

FIG. 1 shows the adhesion inhibition test (i.e. % of cell adhesion vs. concentration) on a vitronectin surface of α_{V}β₃ integrin over-expressed human umbilical vein endothelial cells (HUVEC) by the addition of the compound of example 23 (3), comparative compound cilengitide (C) and two comparative RGD-cyclopentapeptides (1 and 2).
FIG. 2 shows the gene expression patterns of several genes associated with angiogenensis for comparative cilengitide (C), two comparative RGD-cyclopentapeptides (1 and 2), and the compound of the example 23 (3).

### DETAILED DESCRIPTION OF EMBODIMENTS OF THE INVENTION

The present invention relates to compounds of formula cyclo[Arg-Asp-(beta-Lactam)] (I) which are cyclotetrapeptides. In the present invention, the terms Arg, R, and arginine are intended to have the same meaning and are indistinctly used. Further, the terms Asp, D, and aspartic acid are intended to have the same meaning and are indistinctly used. The configuration of the aminoacids R and D in the sequence is L.

Compounds of formula (I) may also be represented as compounds of the formula 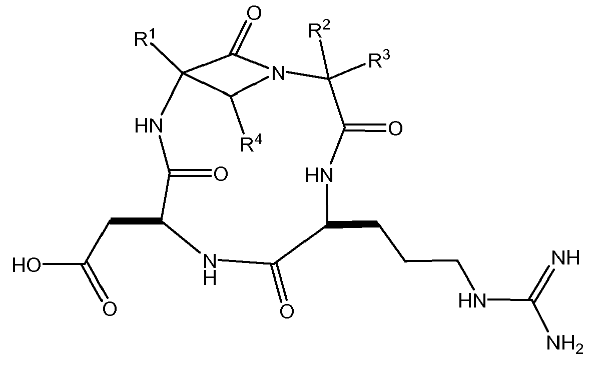 wherein R¹, R², R³ and R⁴ have the meaning previously defined, or as compounds of the formulas:
cyclo[Asp-(beta-Lactam)-Arg]
cyclo[(beta-Lactam)-Arg-Asp]

For the purposes of this invention, the term alkyl means a saturated straight or branched hydrocarbon chain which contains the number of carbon atoms specified in the description or claims. Examples include, among others, the groups methyl, ethyl, n-propyl, isopropyl, n-butyl, n-pentyl, etc. The term haloalkyl refers to a group resulting from the replacement of one or more hydrogen atoms from an alkyl group with one or more halogen atoms, which can be the same or different. Examples include, among others, chloromethyl, trifluoromethyl, 2-bromoethyl, 4-fluoropentyl, etc.

The term alkenyl refers to an unsaturated straight or branched hydrocarbon chain which contains the number of carbon atoms specified in the description or claims, and one or more double bonds. Examples include, among others, ethenyl, 1-propen-1-yl, 1-buten-2-yl, 2-methyl-1-propen-1-yl, 1,3-butadien-1-yl, 2-hexenyl, etc.

The term alkynyl refers to an unsaturated straight or branched hydrocarbon chain which contains which contains the number of carbon atoms specified in the description or claims, and one or more triple bonds. Examples include, among others, ethynyl, 1-propynyl, 2-butynyl, 1,3-butad inyl, 4-pentynyl, 1-hexynyl, etc.

The term (C₃-C₁₀)cycloalkyl refers to a saturated carbocyclic ring system which may be monocyclic or bicyclic and which contains from 3 to 10 carbon atoms. Examples include, among others, cyclopropane, cyclobutane, cyclopentane, cyclohexane, cyclooctane, etc.

The term (C₂-C₉)heterocycloalkyl refers to a (C₃-C₁₀)cycloalkyl ring system as defined above, wherein one or more carbon atoms have been replaced with heteroatoms selected from N, O and S. Examples include, among others, aziridin, oxirane, oxetan, imidazolidine, isothiazolidine, isoxazolidine, oxazolidine, pyrazolidine, pyrrolidine, thiazolidine, dioxane, morpholine, piperazine, piperidine, pyran, tetrahydropyran, azepine, oxazine, oxazoline, pyrroline, thiazoline, pyrazoline, imidazoline, isoxazoline, isothiazoline, etc.

The term (C₆-C₁₂)aryl refers to an aromatic carbocyclic ring system which may contain one or two separated or condensed rings, each containing from 6 to 12 carbon atoms. Examples include phenyl, biphenyl, 1-naphthyl, 2-naphthyl, etc.

The term (C₅-C₁₁)heteroaryl refers to an (C₆-C₁₂)aryl ring system as defined above, wherein one or more carbon atoms have been replaced with heteroatoms selected from N, O and S. Examples include, among others, 1,2,3-triazolyl, 2-pyridyl, 3-furyl, 2-thienyl, 1-pyrrolyl, 2-imidazolyl, 3-pyrazolyl, 2-oxazolyl, 4-isoxazolyl, 2-thiazolyl, 3-isothiazolyl, benzimidazole, benzofuran, benzothiazole, benzothiophene, imidazopyrazine, imidazopyridazine, imidazopyridine, imidazopyrimidine, indazole, indole, isoindole, isoquinoline, naphthiridine, pyrazolopyrazine, pyrazolopyridine, pyrazolopyrimidine, purine, quinazoline, quinoline, quinoxaline, etc.

The term halogen means fluoro, chloro, bromo or iodo.

The expression "optionally substituted with one or more" means that a group can be substituted with one or more, preferably with 1, 2, 3, 4 or 5 substituents, provided that this group has 1, 2, 3, 4 or 5 positions susceptible of being substituted.

The present invention also relates to all the stereoisomers of the compound of formula (I), including enantiomers, diastereoisomers or mixtures thereof.

In the (beta-Lactam) moiety there may exist three chiral centers. For the purposes of the invention, the stereochemistry of the compounds of the invention related to these chiral centers is indicated with the numbers 3, 4 and 1' as shown below:

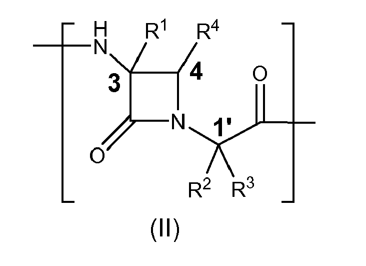

Compounds of formula (I) or any of its stereoisomers can be in form of pharmaceutically acceptable salts. There is no limitation on the type of salt that can be used, provided that these are pharmaceutically acceptable when they are used for therapeutic purposes. The term pharmaceutically acceptable salts embraces salts commonly used to form alkali metal salts and to form addition salts of free acids or free bases.

Salts can be prepared by treating the compound of formula (I) with a desired acid or base to give a salt in the conventional manner. For instance, such compounds can be prepared from the free basic or acid forms with stoichiometric amounts of the appropriate base or acid in water, in an organic solvent, or in their mixtures. In general, non aqueous media are preferred, particularly diethyl ether, ethyl acetate, ethanol, isopropanol or acetonitrile. Examples of salts formed by acid addition include salts of mineral acids such as hydrochloride, hydrobromide, hydroiodide, sulfate, nitrate, phosphate, and salts of organic acids, for example acetate, maleate, fumarate, citrate, oxalate, succinate, tartrate, malate, mandelate, methanesulfonate and p-toluenesulfonate. Examples of salts formed by base addition include salts of inorganic bases, such as sodium, potassium, calcium, ammonium, magnesium, aluminum, lithium, and organic bases, for example, ethylenediamine, ethanolamine, N,N-dialkylethanolamine, triethanolamine and the salts of basic amino acids. In a preferred embodiment, the compounds of formula (I) are in the form of trifluoroacetate salts.

The compounds of formula (I) and their salts may differ in some physical properties but they are equivalent for the purposes of the present invention.

Further, the compounds of the invention may present crystalline forms as free compounds or as solvates, including hydrated forms, all of them being included within the scope of the invention. In general, the solvated forms with pharmaceutically acceptable solvents such as water, ethanol and the like are equivalent to the unsolvated form for the purposes of the invention.

A preferred pharmaceutically acceptable form is crystalline, incorporated as such in a pharmaceutical composition. In the case of salts and solvates, residual solvents and additional ionic compounds have to be nontoxic. The compounds of the invention may present different polymorphic forms, all of them included in the invention.

Cyclotetrapeptides of the formula (I) containing totally or partially derivatized aminoacids also form part of the invention. Derivatized compounds include prodrugs transformable in vivo into the compounds of the invention. Such derivatized compounds are evident for an expert in the art and include, depending on the functional groups presented in the molecule and without limitation, 1,2,3-triazoles, esters, amino acid esters, phosphate esters, sulfonate esters of metal salts, carbamates and amides.

Particularly preferred are the derivatives or prodrugs that increase the bioavailability of the compounds of the invention when such compounds are administered to patients (for example, allowing oral administration to get an easier absorption in blood) or improve the administration of the original compound for a biological behavior with respect to the original species (for instance, in brain or lymphatic system).

In a preferred embodiment, in a compound of formula (I), R¹ is selected from hydrogen, (C₁-C₁₂)alkyl, (C₂-C₁₂)alkenyl, and (C₂-C₁₂)alkynyl; wherein all these groups may be optionally substituted as previously mentioned.

More preferably, R¹ is selected from hydrogen, (C₁-C₁₂)alkyl, (C₁-C₁₂)alkyl substituted with (C₆-C₁₂)aryl or with substituted (C₆-C₁₂)aryl, (C₁-C₁₂)alkyl substituted with (C₅-C₁₁)heteroaryl or with substituted (C₅-C₁₁)heteroaryl, (C₁-C₁₂)alkyl substituted with -COR^{a}, (C₂-C₁₂)alkenyl, and (C₂-C₁₂)alkynyl.

Even more preferably, R¹ is selected from hydrogen, (C₁-C₄)alkyl, (C₁-C₄)alkyl substituted with phenyl or with substituted phenyl, (C₁-C₄)alkyl substituted with triazolyl or with substituted triazolyl, (C₁-C₄)alkyl substituted with -CHO, (C₂-C₄)alkenyl, and (C₂-C₄)alkynyl.

Even more preferably, R¹ is selected from hydrogen; methyl; benzyl; benzyl substituted with one or more halogen atoms; benzyl substituted with one or more halo(C₁-C₄)alkyl groups; 1-(2,3,4,6-tetra-O-benzyl-α-D-mannosyl)-1,2,3-triazol-4-yl]methyl; formylmethyl; allyl; and propargyl.

Even more preferably, R¹ is benzyl, benzyl substituted with one or more halogen atoms, or benzyl substituted with one or more halo(C₁-C₄)alkyl groups. Even more preferably, R¹ is benzyl, 3-trifluoromethylbenzyl or 2,3,4,5,6-pentafluorobenzyl. In the most preferred embodiment, R¹ is benzyl.

In another preferred embodiment, in a compound of formula (I), R² is selected from hydrogen, (C₁-C₁₂)alkyl, and (C₆-C₁₂)aryl, wherein all these groups may be optionally substituted as previously mentioned. More preferably, R² is selected from hydrogen, optionally substituted (C₁-C₄)alkyl, and optionally substituted phenyl. More preferably, R² is selected from hydrogen, (C₁-C₄)alkyl, and phenyl. In the most preferred embodiment, R² is methyl.

In another preferred embodiment, in a compound of formula (I), R³ is hydrogen, or optionally substituted (C₁-C₁₂)alkyl. More preferably, R³ is hydrogen, or optionally substituted (C₁-C₄)alkyl. More preferably, R³ is hydrogen, or (C₁-C₄)alkyl. In the most preferred embodiment, R³ is methyl.

In another preferred embodiment, in a compound of formula (I), R² and R³ form together an unsubstituted 3- to 5-membered saturated monocyclic carbocyclic ring.

In another preferred embodiment, in a compound of formula (I), R⁴ is hydrogen, or optionally substituted (C₂-C₉)heterocycloalkyl. More preferably, R⁴ is hydrogen, or (C₂-C₉)heterocycloalkyl substituted with one or more (C₁-C₄)alkyl groups. More preferably, R⁴ is hydrogen, or (4S)-2,2-dimethyl-1,3-dioxolan-4-yl. In the most preferred embodiment, R⁴ is hydrogen.

In another preferred embodiment, R¹ is selected from benzyl, benzyl substituted with one or more halogen atoms, and benzyl substituted with one or more halo(C₁-C₄)alkyl groups; R² is selected from hydrogen, (C₁-C₄)alkyl, and phenyl; R³ is hydrogen, or (C₁-C₄)alkyl; and R⁴ is hydrogen, or (C₂-C₉)heterocycloalkyl substituted with one or more (C₁-C₄)alkyl groups.

In another preferred embodiment, R¹ is selected from benzyl, benzyl substituted with one or more halogen atoms, and benzyl substituted with one or more halo(C₁-C₄)alkyl groups; R² and R³ form together an unsubstituted 3-to 5-membered saturated monocyclic carbocyclic ring; and R⁴ is hydrogen, or (C₂-C₉)heterocycloalkyl substituted with one or more (C₁-C₄)alkyl groups.

Furthermore, all possible combinations of the above-mentioned embodiments form also part of this invention.

In another preferred embodiment of the invention, the compound of formula (I) is selected from the group consisting of:
Compound (I) wherein R¹ = CH₂Ph; R² = R³ = R⁴ = H (3S configuration);
Compound (I) wherein R¹ = CH₂Ph; R² = Ph, R³ = R⁴= H (3S, 1'R configuration);
Compound (I) wherein R¹ = CH₂Ph; R² = Ph, R³ = R⁴= H (3S, 1'S configuration);
Compound (I) wherein R¹ = CH₂Ph; R² = methyl, R³ = R⁴= H (3S, 1'S configuration);
Compound (I) wherein R¹ = CH₂Ph; R² = methyl, R³ = R⁴= H (3S, 1'R configuration);
Compound (I) wherein R¹ = 3-trifluoromethylbenzyl; R² = R³ = R⁴ = H (3S configuration);
Compound (I) wherein R¹ = methyl; R² = R³ = R⁴ = H (3S configuration); Compound (I) wherein R¹ = methyl; R² = isopropyl; R³ = R⁴ = H (3R, (1'R configuration);
Compound (I) wherein R¹ = CH₂Ph; R² = R³ = methyl; R⁴ = H (3S configuration);
Compound (I) wherein R¹ = CH₂Ph; R² = R³ = together form a cyclopentyl ring; R⁴ = H (3S configuration);
Compound (I) wherein R¹= CH₂C₆F₅; R² = R³ = methyl; R⁴ = H (3S configuration);
Compound (I) wherein R¹= allyl; R² = R³ = methyl; R⁴ = H (3S configuration); Compound (I) wherein R¹ = CH₂CHO; R² = R³ = methyl; R⁴ = H (3S configuration);
Compound (I) wherein R¹= propargyl; R² = R³ = methyl; R⁴ = H (3S configuration);
Compound (I) wherein R¹ = [1-(2,3,4,6-tetra-O-benzyl-α-D-mannosyl)-1,2,3-triazol-4-yl]methyl; R² = R³ = methyl; R⁴ = H (3S configuration);
Compound (I) wherein R¹ = R² = R³ = H; R⁴ = (4S)-2,2-dimethyl-1,3-dioxolan-4-yl (3S 4R configuration); or
Compound (I) wherein R¹ = R² = R³ = H; R⁴ = (4S)-2,2-dimethyl-1,3-dioxolan-4-yl (3R 4R configuration);

The compounds of formula (I) can be conveniently prepared by reacting a compound of formula H-Z-OH (III) with a condensing agent, optionally in the presence of a base, wherein Z is a biradical selected from -Arg(R⁵)-Asp(R⁶)-(beta-Lactam)-, -Asp(R⁶)-(beta-Lactam)-Arg(R⁵)-, and -(beta-Lactam)-Arg(R⁵)-Asp(R⁶)-; R⁵ is H or an amino protecting group PG¹; R⁶ is OH or a carboxyl protecting group PG²; and (beta-Lactam) is a biradical of the formula (II) as defined above.

The amino and carboxyl protecting groups mentioned above can be defined as chemical fragments able to prevent (block) the modification of the amino and the carboxy groups respectively, in most chemical reactions, but easily removable under certain conditions after a transformation in a different position of the molecule. Preferred amino protecting groups are tert-butoxycarbonyl (Boc), tert-allyloxycarbonyl (Alloc), 9-fluorenylmethoxycarbonyl (Fmoc), 9-fluorenylmethyl (Fm), benzyloxycarbonyl (Cbz), methoxycarbonyl, ethoxycarbonyl, benzyl (Bn), 2,2,4,6,7-pentamethyldihydrobenzofuran-5-sulfonyl (Pbf), acetyl, benzoyl, C₆H₅-SO₂-, 4-NO₂C₆H₄-SO₂-, 2-NO₂C₆H₄-SO₂-, 2,4-(NO₂)₂C₆H₃-SO₂-, CHO-, Me₃Si- or ^{t}BuMe₂Si-. Preferred carboxyl protecting groups are O(C₁-C₄)alkyl, such as methoxy or tert-butoxy. Other preferred carboxyl protecting groups are OBn, OFm, OC₆F₅ and SC₆H₅.

The cyclization of H-Z-OH (III) can be conducted using peptide synthesis reagents well-known in the art, and if necessary the protecting groups can be cleaved in a later step of the synthesis applying conventional methods also well-known in the art.

Thus, the cyclization can be carried out with a condensing agent, such as 1-hydroxy-7-azabenzotriazole (HOAT) and O-(7-azabenzotriazol-1-yl)-N,N,N',N'-tetramethyluronium hexafluorophosphate (HATU), HATU, 2-ethoxy-1-ethoxycarbonyl-1,2-dihydroquinoline (EEDQ), or 1-ethyl-3-(3-dimethylaminopropyl)carbodiimide (EDC) and 1-hydroxy-benzotriazole (HOBT) in the presence of a base, such as KHCO₃ or triethylamine (TEA), in a suitable solvent such as N,N-dimethylformamide (DMF), or CH₂Cl₂, at a suitable temperature, preferably between -15°C and room temperature.

The intermediates of formula H-Z-OH (III) or their pharmaceutically acceptable salts, also form part of the present invention. Compounds of formula (III) include:
H-Arg(R⁵)-Asp(R⁶)-(beta-Lactam)-OH (IIIa)
H-Asp(R⁶)-(beta-Lactam)-Arg(R⁵)-OH (IIIb)
H-(beta-Lactam)-Arg(R⁵)-Asp(R⁶)-OH (IIIc)
wherein R⁵, R⁶ and (beta-Lactam) are as previously defined.

For the purposes of the invention, the biradicals -Arg(R⁵)-, and -Asp(R⁶)-, are meant to be respectively: 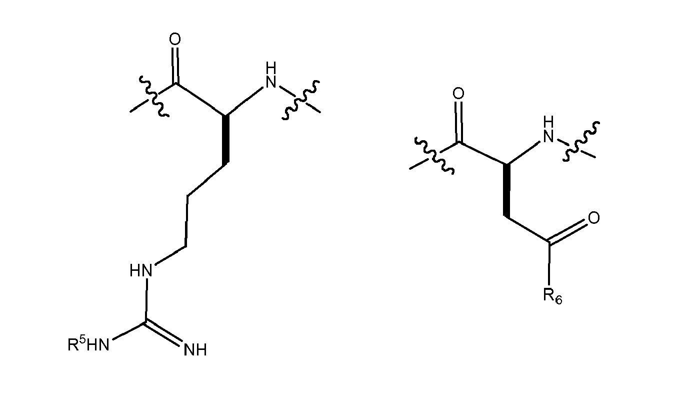 wherein R⁵ and R⁶ are as previously defined.

In a preferred embodiment, in a compound of formula (III), R¹ is selected from hydrogen, (C₁-C₁₂)alkyl, (C₂-C₁₂)alkenyl, and (C₂-C₁₂)alkynyl; wherein all these groups may be optionally substituted as previously mentioned. More preferably, R¹ is selected from hydrogen, (C₁-C₁₂)alkyl, (C₁-C₁₂)alkyl substituted with (C₆-C₁₂)aryl or with substituted (C₆-C₁₂)aryl, (C₁-C₁₂)alkyl substituted with (C₅-C₁₁)heteroaryl or with substituted (C₅-C₁₁)heteroaryl, (C₁-C₁₂)alkyl substituted with -COR^{a}, (C₂-C₁₂)alkenyl, and (C₂-C₁₂)alkynyl. Even more preferably, R¹ is selected from hydrogen, (C₁-C₄)alkyl, (C₁-C₄)alkyl substituted with phenyl or with substituted phenyl, (C₁-C₄)alkyl substituted with triazolyl or with substituted triazolyl, (C₁-C₄)alkyl substituted with -CHO, (C₂-C₄)alkenyl, and (C₂-C₄)alkynyl. Even more preferably, R¹ is selected from hydrogen; methyl; benzyl; benzyl substituted with one or more halogen atoms; benzyl substituted with one or more halo(C₁-C₄)alkyl groups; 1-(2,3,4,6-tetra-O-benzyl-α-D-mannosyl)-1,2,3-triazol-4-yl]methyl; formylmethyl; allyl; and propargyl. Even more preferably, R¹ is benzyl, benzyl substituted with one or more halogen atoms, or benzyl substituted with one or more halo(C₁-C₄)alkyl groups. Even more preferably, R¹ is benzyl, 3-trifluoromethylbenzyl or 2,3,4,5,6-pentafluorobenzyl. In the most preferred embodiment, R¹ is benzyl.

In another preferred embodiment, in a compound of formula (III), R² is selected from hydrogen, (C₁-C₁₂)alkyl, and (C₆-C₁₂)aryl, wherein all these groups may be optionally substituted as previously mentioned. More preferably, R² is selected from hydrogen, optionally substituted (C₁-C₄)alkyl, and optionally substituted phenyl. More preferably, R² is selected from hydrogen, (C₁-C₄)alkyl, and phenyl. In the most preferred embodiment, R² is methyl.

In another preferred embodiment, in a compound of formula (III), R³ is hydrogen, or optionally substituted (C₁-C₁₂)alkyl. More preferably, R³ is hydrogen, or optionally substituted (C₁-C₄)alkyl. More preferably, R³ is hydrogen, or (C₁-C₄)alkyl. In the most preferred embodiment, R³ is methyl.

In another preferred embodiment, in a compound of formula (III), R² and R³ form together an unsubstituted 3- to 5-membered saturated monocyclic carbocyclic ring.

In another preferred embodiment, in a compound of formula (III), R⁴ is hydrogen, or optionally substituted (C₂-C₉)heterocycloalkyl. More preferably, R⁴ is hydrogen, or (C₂-C₉)heterocycloalkyl substituted with one or more (C₁-C₄)alkyl groups. More preferably, R⁴ is hydrogen, or (4S)-2,2-dimethyl-1,3-dioxolan-4-yl. In the most preferred embodiment, R⁴ is hydrogen.

In another preferred embodiment, in a compound of formula (III), R⁵ is 2,2,4,6,7-pentamethyldihydrobenzofuran-5-sulfonyl (Pbf). In another preferred embodiment, in a compound of formula (III), R⁶ is tert-butoxy.

In another preferred embodiment, in a compound of formula (III), R¹ is selected from benzyl, benzyl substituted with one or more halogen atoms, and benzyl substituted with one or more halo(C₁-C₄)alkyl groups; R² is selected from hydrogen, (C₁-C₄)alkyl, and phenyl; R³ is hydrogen, or (C₁-C₄)alkyl; R⁴ is hydrogen, or (C₂-C₉)heterocycloalkyl substituted with one or more (C₁-C₄)al kyl groups; R⁵ is 2,2,4,6,7-pentamethyldihydrobenzofuran-5-sulfonyl (Pbf); and R⁶ is tert-butoxy.

In another preferred embodiment, in a compound of formula (III), R¹ is selected from benzyl, benzyl substituted with one or more halogen atoms, and benzyl substituted with one or more halo(C₁-C₄)alkyl groups; R² and R³ form together an unsubstituted 3- to 5-membered saturated monocyclic carbocyclic ring; R⁴ is hydrogen, or (C₂-C₉)heterocycloalkyl substituted with one or more (C₁-C₄)alkyl groups; R⁵ is 2,2,4,6,7-pentamethyldihydrobenzofuran-5-sulfonyl (Pbf); and R⁶ is tert-butoxy.

The cyclization of a compound of formula H-Z-OH (III), wherein Z is a biradical selected from -Arg(R⁵)-Asp(R⁶)-(beta-Lactam)-, -Asp(R⁶)-(betaLactam)-Arg(R⁵)-, and -(beta-Lactam)-Arg(R⁵)-Asp(R⁶)-; R⁵ is PG¹ and/or R⁶ is PG², gives rise to a compound of formula (I')
cyclo[Arg(R⁵)-Asp(R⁶)-(beta-Lactam)] (I')
wherein R⁵ is PG¹ and/or R⁶ is PG². After removing these protecting groups of a compound of formula (I') by conventional methods, the corresponding compound of formula (I) is obtained.

The compounds of formula (I') also form part of the invention. In a preferred embodiment, in a compound of formula (I'), R¹ is selected from hydrogen, (C₁-C₁₂)alkyl, (C₂-C₁₂)alkenyl, and (C₂-C₁₂)alkynyl; wherein all these groups may be optionally substituted as previously mentioned. More preferably, R¹ is selected from hydrogen, (C₁-C₁₂)alkyl, (C₁-C₁₂)alkyl substituted with (C₆-C₁₂)aryl or with substituted (C₆-C₁₂)aryl, (C₁-C₁₂)alkyl substituted with (C₅-C₁₁)heteroaryl or with substituted (C₅-C₁₁)heteroaryl, (C₁-C₁₂)alkyl substituted with -COR^{a}, (C₂-C₁₂)alkenyl, and (C₂-C₁₂)alkynyl. Even more preferably, R¹ is selected from hydrogen, (C₁-C₄)alkyl, (C₁-C₄)alkyl substituted with phenyl or with substituted phenyl, (C₁-C₄)alkyl substituted with triazolyl or with substituted triazolyl, (C₁-C₄)alkyl substituted with -CHO, (C₂-C₄)alkenyl, and (C₂-C₄)alkynyl. Even more preferably, R¹ is selected from hydrogen; methyl; benzyl; benzyl substituted with one or more halogen atoms; benzyl substituted with one or more halo(C₁-C₄)alkyl groups; 1-(2,3,4,6-tetra-O-benzyl-α-D-mannosyl)-1,2,3-triazol-4-yl]methyl; formylmethyl; allyl; and propargyl. Even more preferably, R¹ is benzyl, benzyl substituted with one or more halogen atoms, or benzyl substituted with one or more halo(C₁-C₄)alkyl groups. Even more preferably, R¹ is benzyl, 3-trifluoromethylbenzyl or 2,3,4,5,6-pentafluorobenzyl. In the most preferred embodiment, R¹ is benzyl.

In another preferred embodiment, in a compound of formula (I'), R² is selected from hydrogen, (C₁-C₁₂)alkyl, and (C₆-C₁₂)aryl, wherein all these groups may be optionally substituted as previously mentioned. More preferably, R² is selected from hydrogen, optionally substituted (C₁-C₄)alkyl, and optionally substituted phenyl. More preferably, R² is selected from hydrogen, (C₁-C₄)alkyl, and phenyl. In the most preferred embodiment, R² is methyl.

In another preferred embodiment, in a compound of formula (I'), R³ is hydrogen, or optionally substituted (C₁-C₁₂)alkyl. More preferably, R³ is hydrogen, or optionally substituted (C₁-C₄)alkyl. More preferably, R³ is hydrogen, or (C₁-C₄)alkyl. In the most preferred embodiment, R³ is methyl.

In another preferred embodiment, in a compound of formula (I'), R² and R³ form together an unsubstituted 3- to 5-membered saturated monocyclic carbocyclic ring.

In another preferred embodiment, in a compound of formula (I'), R⁴ is hydrogen, or optionally substituted (C₂-C₉)heterocycloalkyl. More preferably, R⁴ is hydrogen, or (C₂-C₉)heterocycloalkyl substituted with one or more (C₁-C₄)alkyl groups. More preferably, R⁴ is hydrogen, or (4S)-2,2-dimethyl-1,3-dioxolan-4-yl. In the most preferred embodiment, R⁴ is hydrogen.

In another preferred embodiment, in a compound of formula (I'), R⁵ is 2,2,4,6,7-pentamethyldihydrobenzofuran-5-sulfonyl (Pbf). In another preferred embodiment, in a compound of formula (III), R⁶ is tert-butoxy.

In another preferred embodiment, in a compound of formula (I'), R¹ is selected from benzyl, benzyl substituted with one or more halogen atoms, and benzyl substituted with one or more halo(C₁-C₄)alkyl groups; R² is selected from hydrogen, (C₁-C₄)alkyl, and phenyl; R³ is hydrogen, or (C₁-C₄)alkyl; R⁴ is hydrogen, or (C₂-C₉)heterocycloalkyl substituted with one or more (C₁-C₄)alkyl groups; R⁵ is 2,2,4,6,7-pentamethyldihydrobenzofuran-5-sulfonyl (Pbf); and R⁶ is tert-butoxy.

In another preferred embodiment, in a compound of formula (I'), R¹ is selected from benzyl, benzyl substituted with one or more halogen atoms, and benzyl substituted with one or more halo(C₁-C₄)alkyl groups; R² and R³ form together an unsubstituted 3- to 5-membered saturated monocyclic carbocyclic ring; R⁴ is hydrogen, or (C₂-C₉)heterocycloalkyl substituted with one or more

(C₁-C₄)alkyl groups; R⁵ is 2,2,4,6,7-pentamethyldihydrobenzofuran-5-sulfonyl (Pbf); and R⁶ is tert-butoxy.

In general, the intermediates of formula H-Z-OH (III) can be prepared by known methods, for example the above-mentioned methods of peptide synthesis and of elimination of protecting groups. For example, they can be obtained by deprotection of the corresponding protected tetrapeptides of formula PG¹-Z-PG² (III'), wherein Z, the amino protecting group PG¹, and the carboxyl protecting group PG² are as previously defined.

Generally, the groups Boc and O^{t}Bu can be removed, for example, preferably using trifluoroacetic acid (TFA) in dichloromethane or with HCl in dioxane, while the Fmoc group can be eliminated with a solution of dimethylamine, diethylamine or piperidine in dimethylformamide (DMF). Protecting groups which can be removed by hydrogenolysis, such as Cbz or benzyl, can be eliminated, for example, by treatment with hydrogen in the presence of a catalyst such as palladium, preferably on charcoal, in a suitable solvent, such as methanol, ethanol, or dimethylformamide (DMF), at a temperature between 0-100°C and at pressures of between 1-200 bar.

The intermediates of formula PG¹-Z-PG² (III') can be prepared from the corresponding deprotected peptides.

Moreover, the intermediates of formula PG¹-Z-PG² (III') can be prepared by coupling a compound of formula (IV) with a compound of formula (V);
R⁷-Y-R⁸ (IV) R⁹-Q-R¹⁰ (V)
wherein Y is a biradical selected from -Arg(R⁵)-Asp(R⁶)-, -Asp(R⁶)-(beta-Lactam)-, and -(beta-Lactam)-Arg(R⁵)-;
Q is a biradical selected from and -Arg(R⁵)-, -Asp(R⁶)-, and -(beta-Lactam)-; R⁷ is hydrogen or PG¹;
R⁸ is X when R⁷ is PG¹; and R⁸ is PG² when R⁷ is hydrogen;
R⁹ is hydrogen when R⁷ is PG¹; and R⁹ is PG¹ when R⁷ is hydrogen;
R¹⁰ is X when R⁹ is PG¹; and R¹⁰ is PG² when R⁹ is hydrogen;
X is selected from OH, OLi, ONa, OK, F, and Cl; and
the (beta-Lactam), R⁵, R⁶, PG¹, and PG² are as previously defined; with the condition that Y and Q comprise different aminoacids.

In particular, compounds of formula (IV) encompass:
R⁷-Arg(R⁵)-Asp(R⁶)-R⁸ (IVa)
R⁷-Asp(R⁶)-(beta-Lactam)-R⁸ (IVb)
R⁷-(beta-Lactam)-Arg(R⁵)-R⁸ (IVc)
and compounds of formula (V) encompass:
R⁹-(beta-Lactam)-R¹⁰(Va)
R⁹-Arg(R⁵)-R¹⁰ (Vb)
R⁹-Asp(R⁶)-R¹⁰ (Vc)

Thus, the intermediates of formula PG¹-Z-PG² (III') can be prepared by coupling a compound of formula (IVa) with a compound of formula (Va); or a compound of formula (IVb) with a compound of formula (Vb); or a compound of formula (IVc) with a compound of formula (Vc), wherein the meanings of the variables are as previously defined.

If desired, the starting substances (IV) and (V) can also be formed in situ, so that they are not isolated from the reaction mixture but are immediately reacted further to give the compounds of the formula (III).

When in any the compounds of formula (IV) or (V) above, R⁸ or R¹⁰ is X, and X is OH, OLi, ONa, OK, the reaction can be carried out with a condensing agent, optionally in the presence of a base as previously specified. When X is F, and Cl, the reaction can be carried out for instance in pyridine or in the presence of a base such as triethylamine in a suitable solvent such as dichloromethane, and at a suitable temperature, preferably at 0°C.

In its turn, the intermediates of formula (IV) and (V) wherein R⁷ or R⁹ and/or R⁸ or R¹⁰ is H can be obtained by deprotection of the corresponding protected compounds of formula PG¹-Y-PG² (IV') and PG¹-Q-PG² (V') respectively. In the case of compounds of formula (IV) and (V) wherein R⁸ or R¹⁰ is X, the deprotection gives rise to compounds of formula (IV) and (V) wherein X is OH, and if desired, this group can be transformed into another X group by conventional methods.

The intermediates of formulas PG¹-Y-PG² (IV') and PG¹-Q-PG² (V') can be prepared from the corresponding deprotected peptides.

Moreover, the intermediates of formula PG¹-Y-PG² (IV') can be prepared by coupling a compound of formula (V) with a compound of formula (VI);
R⁷-Q-R⁸ (V) R⁹-Q'-R¹⁰ (VI)
wherein Q' is a biradical selected from and -Arg(R⁵)-, -Asp(R⁶)-, and -(beta-Lactam)-; and Q, R⁷, R⁸, R⁹ and R¹⁰ are as previously defined; with the condition that Q and Q' comprise different aminoacids.

If desired, the starting substances (V) and (VI) can also be formed in situ, so that they are not isolated from the reaction mixture but are immediately reacted further to give the compounds of the formula (IV').

These reactions can be carried out as specified above. Peptide coupling can also be carried out by solid phase synthesis.

The compounds of formula (V) of formula (VI), wherein Q is -(beta-Lactam)-wherein R¹, R², R³, R⁴, R⁷, and R⁸ are as previously defined; can be prepared as described in Angew. Chem. Int. Ed. 1999, vol. 38, pp. 3056-3058, J. Org. Chem. 2001, vol. 66, pp. 6333-6338, J. Am. Chem. Soc. 2003, vol. 125, pp. 16243-16260, or Org. Lett. 2007, vol. 9, pp. 101-104.

Reaction products can be purified, if necessary, by conventional methods such as crystallization, chromatography or trituration. When the processs described above to obtain the compounds of the invention afford stereoisomer mixtures, they can be used as such, or they can be separated by conventional methods like preparative chromatography. In case stereocenters are present, compounds can be prepared in racemic form; alternatively, individual enantiomers can be prepared by enantioselective synthesis or by resolution.

The present invention also relates to a pharmaceutical composition comprising a compound of formula (I) or a pharmaceutically acceptable salt thereof, or a derivative thereof, or a prodrug thereof or a stereoisomer thereof, together with excipients or other auxiliary agents, including adjuvants, if necessary. The election of the pharmaceutical formulation will depend upon the nature of the active compound and its route of administration. Any route of administration may be used, for example oral, parenteral and topical administration.

For example, the pharmaceutical composition may be formulated for oral administration and may contain one or more physiologically compatible carriers or excipients, in solid or liquid form. These preparations may contain conventional ingredients such as binding agents, fillers, lubricants, and acceptable wetting agents.

The pharmaceutical composition may be formulated for parenteral administration in combination with conventional injectable liquid carriers, such as water or suitable alcohols. Conventional pharmaceutical excipients for injection, such as stabilizing agents, solubilizing agents, and buffers, may be included in such compositions. These pharmaceutical compositions may preferably be injected intramuscularly, intraperitoneally, or intravenously.

The pharmaceutical compositions may be in any form, including, among others, tablets, pellets, capsules, aqueous or oily solutions, suspensions, emulsions, or dry powdered forms suitable for reconstitution with water or other suitable liquid medium before use, for immediate or retarded release.

The specific dose of the compound of the invention to obtain a therapeutic benefit may vary depending on the particular circumstances of the individual patient including, among others, the size, weight, age and sex of the patient, the nature and stage of the disease, the aggressiveness of the disease, and the route of administration. For example, a daily dosage of from about 0.01 to about 100 mg/kg/day may be used.

According to H. Kessler et al, J. Biol. Chem. 1994, vol. 269, pp. 20233-20238, in RGD cyclopentapetides of the Cilengitide type, the distance (r_{b}) between the pharmacophoric points, i.e. the distance between the C_{β} atoms of the Arg and Asp residues, is critical for the adhesion of the peptide to the α_{V}β₃ integrin receptor. In particular, a distance of 0.67nm is optimal for interaction with the α_{V}β₃ integrin receptor, a distance of 0.7nm or greater decreases the affinity, and a distance of less than 0.6 nm renders the compound inactive. In the compounds of the invention of formula (I), the (r_{b}) distance is 0.54 nm (5.4 A). However, it has been surprisingly found that, despite of the teaching of the prior art, the affinity for the receptor is maintained as it will be shown in the examples.

Further, the examples will also show that the compounds of formula (I) of the invention have pro-angiogenic properties, and thus behave as α_{V}β₃ integrin agonists. In particular, it has been demonstrated that the compounds of formula (I) do not inhibit the expression of some genes associated with angiogenesis. In fact, the gene expression pattern of the compounds of formula (I) the invention in respect of these genes is opposite to the gene expression pattern shown by the RGD-cyclopentapeptides of the prior art.

Unless otherwise stated, the invention also includes compounds containing one or several isotopically enriched atoms, and their use as markers for diagnostic. Compounds defined as above, excepting those replacing hydrogen by deuterium or tritium, are inside the scope of the invention; for instance, the compounds with carbon atoms enriched by ¹³C or ¹⁴C, or with nitrogen atoms enriched by ¹⁵N.

The present invention also relates to the use of the compounds of formula (I) as radiolabeled imaging agents for noninvasive imaging of integrin expression or as a thrombous imaging agent. The radionuclid choiced is ^{99m}Tc, the radiolabeled RD agent can be used in detection of deep vein thrombosis, hemodinamics of coagulation cascade, visualization of inefective endocarditis, myocardial microthromboemboli, acute cerebral ischemia and as a tumor tracer.

The present invention also relates to the use of the compounds of formula (I) as delivery agents of cytotoxins, liposomes, genes and fluorescent agents to tissues; or as ligands in the surface coating of materials in implant surgery as well as in cell culturing for tissue engineering; or as coatings for growth and physiological functioning of the cells on the biomaterial making up the tissue or organ that is to be replaced.

As mentioned above, the invention also relates to the use of the cyclotetrapeptides of formula (I) for the purification of integrins and in the analysis of integrins, including detection, and/or quantification, and/or separation of integrins. The analysis of integrins may allow the early diagnostic of integrin mediated diseases.

For the above-mentioned purposes, the cyclotetrapeptides of formula (I) are immobilized onto different materials, preferably onto polymers, proteins, chromatographic stationary phases, or fluorescent dyes. These materials also form part of the invention.

The coupling of the cyclotetrapeptides of formula (I) to these materials may take place through the functional groups of the aminoacid side chains. Alternatively, the cyclotetrapeptides of formula (I) may be firstly derivatized, for example introducing functional groups in the substituents R² and/or R³, and subsequently immobilized onto the mentioned materials.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

Acronyms of reagents or techniques used are defined as follows:

| | |
|---|---|
| Arg | Arginine |
| Asp | Aspartic acid |
| Bn | Benzyl |
| Cbz | benzyloxycarbonyl |
| DABCO | 1,4-Diazabicyclo[2.2.2]octane |
| BAIB | bis(acetoxy)iodo-benzene |
| DAPI | 4,6-Diamidine-2-phenylindole |
| DMEM | Dulbecco modified Eagle medium |
| DMF | N,N-Dimethylformamide |
| DMSO | Dimethylsulfoxide |
| EDC | 1-Ethyl-3-(3-dimethylaminopropyl)carbodiimide |
| EEDQ | 2-Ethoxy-1-ethoxycarbonyl-1,2-dihydroquinoline |
| EtOAc | Ethyl acetate |
| HATU | O-(7-Azabenzotriazol-1-yl)-N,N,N',N'-tetramethyluronium |
| hexafluorophosphate | |
| HOAT | 1-Hydroxy-7-azabenzotriazole |
| HOBT | 1-Hydroxy-benzotriazole |
| Hex | Hexane |
| LHMDS | Lithium bis(trimethylsilyl)amide |
| MeCN | Acetonitrile |
| MeOH | Methanol |
| MS (ESI) | Mass spectrometry (Electrospray ionization) |
| o-NsCl | o-Nosyl chloride, 2-nitrobenezenesulfonyl chloride |
| Pbf | 2,2,4,6,7-pentamethyldihydrobenzofuran-5-sulfonyl (Pbf) |
| TEA | Triethylamine |
| TEMPO | 2,2,6,6-tetramethyl-1-piperidinyloxyl |
| THF | Tetrahydrofuran |

### EXAMPLE 1: Preparation of (S)-2-[3-benzyl-3-(2-nitrobenzenesulfonyl)amino-2-oxoazetidin-1-yl]acetic acid (compound of formula (Va): R¹ = CH₂Ph: R²= R³ =R⁴= H; R⁹= 2-nitrobenzenesulfonyl, R¹⁰ = OH; 3S configuration)

A suspension of (S)-a-benzylserine methyl ester (2.1 g, 10 mmol), 2-nitrobenzenesulfonyl chloride (2.2 g, 38 mmol) and KHCO₃ (5.0 g, 50 mmol) in acetonitrile (80 mL) was refluxed over 16 h. To the "in situ" formed (S)-2-benzyl-2-methoxycarbonyl-1-(2-nitrobenzenesulfonyl)aziridine and excess KHCO₃ cooled to 20 °C, was added 2-(O-tert-butyldimethylsilyloxy)-ethylamine (1.75 g, 10 mmol) and the mixture was stirred for 20 h at room temperature. To the resulting suspension was added a saturated aqueous solution of NaHCO₃ (50 mL) and the mixture was extracted with EtOAc (3 x 25 mL). Combined organic phases were dried (MgSO₄) and evaporated under reduced pressure to afford the intermediate. The crude product was purified by column chromatography (silicagel-60, eluent EtOAc/Hex 1:4) yielding methyl (S)-3-(2-tert-butyldimethylsilyloxyethylamino)-2-benzyl-2-(2-nitrobenzenesulfonylamino)propanoate (3.75 g 68%). To a solution of this compound in anhydrous THF (30 mL), cooled at 0 °C, LHMDS (1 M in THF) (25 mL, 25 mmol) was added drop by drop in 5 min and the resulting mixture was stirred, at room temperature, for 6 h. Upon completion an aqueous solution of NaHCO₃ (sat.) (20 mL) was added and the mixture was extracted with CH₂Cl₂ (3 x 15 mL). The organic phase was decanted and dried (MgSO₄). The evaporation under reduced pressure to afford the pure (S)-3-benzyl-3-(2-nitrobenzenesulfonyl-amino)-1-(2-tert-butyldimethylsilyloxyethyl)-azetidin-2-one (3.43 g, 97%). The crude was dissolved in acetone (50 mL). Over this solution, cooled at 0°C, were added, drop by drop, a water (9 mL) solution of chromium trioxide (3.3 g, 33 mmol), sulfuric acid (2.86 mL) and hydrofluoric acid (48%) (0.8 mL, 8 mmol). The resulting solution was stirred, at room temperature, for 1 h. Then, isopropanol (5 mL) and water (100 mL) were successively added. The aqueous phase was extracted with EtOAc (3 x 20 mL), dried (Na₂SO₄) and evaporated. The crude product was purified by column chromatography (silicagel-60, eluent hexane/EtOAc 1:5). Overall yield: 2.43 g (58%).
[α]²⁰D = -2.5 (c = 0.075, MeOH); ¹H NMR (500MHz, CD₃OD, δ): 7.77-8.12 (m, 4H, arom.), 7.31-7.21 (m, 5H, arom.), 3.67 (d, 1 H, NCH₂, β-Lactam, J= 6.0 Hz), 3.59 (s, 2H, PhCH₂CO₂H), 3.56 (d, 1 H, NCH₂, β-Lactam, J= 6.1 Hz), 3.28 (dd, 2H, CH₂Ph).

### EXAMPLE 2: Preparation of (R)-2-[3(S)-benzyl-3-(2-nitrobenzenesulfonyl)amino-2-oxoazetidin-1-yl]-2-phenylacetic acid (compound of formula (Va): R¹ = CH₂Ph; R² = Ph, R³ = R⁴= H; R⁹ = 2-nitrobenzenesulfonyl, R¹⁰ = OH; 3S, 1'R configuration)

The process of example 1 was followed, starting from (S)-a-benzylserine (2.1 g, 10 mmol), and (R)-2-(O-tert-butyldimethylsilyloxy)-1-phenyl-ethylamine (2.51 g, 10 mmol). Overall yield: 2.18 g (44%).

Mp = 168-170°C; [α]²⁰D = -42.8 (c = 0.75, MeOH). IR (cm⁻¹, KBr): 3366 (NH), 1763, 1643 (C=O), 1547, 1410 (NO₂). ¹H NMR (500 MHz, CD₃OD): δ 7.80-7.55 (m, 4H), 7.36-7.22 (m, 10H), 5.31 (s, 1 H), 3.75 (d, 1 H, J = 5.8 Hz), 3.28 (d, 1 H, J = 5.7 Hz), 3.26 (br s, 2H). ¹³C NMR (75 MHz, CD₃OD): δ 170.6, 165.6, 147.7, 134.8, 134.5, 134.0, 132.6, 130.3, 128.8, 128.4, 127.2, 125.0, 69.5, 57.9, 51.3, 40.3. MS m/z (Ion Source Type: ESI, negative polarity): MS-1: 494.1; MS2(494.1): 345.6, 344.1, 301.6, 271.7; MS3(344.0): 301.8, 300.9, 271.9, 270.9, 219.0, 195.9, 183.9, 170.8, 145.9, 122.0 Anal. calcd. for C₂₄H₂₁N₃O₇S: C, 58.17; H, 4.27; N, 8.48. Found: C, 58.46; H, 4.08; N, 8.22.

### EXAMPLE 3: Preparation of (S)-2-[3(S)-benzyl-3-(2-nitrobenzenesulfonyl)amino-2-oxoazetidin-1-yl]-2-phenylacetic acid (compound of formula (Va): R¹ = CH₂Ph: R² = Ph, R³ = R⁴ = H; R⁹ = 2-nitrobenzenesulfonyl, R¹⁰ = OH; 3S, 1'S configuration)

The process of example 1 was followed, starting from (S)-α-benzylserine (2.1 g, 10 mmol), and (S)-2-(O-tert-butyldimethylsilyloxy)-1-phenyl-ethylamine (2.51 g, 10 mmol). Overall yield: 2.58 g (52%).

Mp = 194-196°C; [α]²⁰_{D} = +4.8 (c = 0.5, MeOH). IR (cm⁻¹, KBr): 3442 (NH), 1742, 1715 (C=O), 1530 (NO₂). ¹H NMR (CD₃OD, 500 MHz): δ 7.89-7.70 (m, 4H), 7.20-6.74 (m, 10H), 5.07 (s, 1 H), 3.70 (d, 1 H, J = 6.3 Hz), 3.11 (d, 1 H, J = 6.3 Hz), 3.13 (br s, 2H). ¹³C NMR (75 MHz, CD₃OD): δ 173.8, 166.8, 147.9, 135.7, 134.6, 133.1, 132.3, 130.4, 130.2, 128.0, 127.9, 127.7, 126.9, 126.8, 69.7, 60.3, 49.7, 39.1. MS m/z (Ion Source Type: ESI, negative polarity): MS-1: 494.0; MS2(494.1): 345.6, 344.1, 301.5, 271.7; MS3(344.1): 301.9, 300.9, 270.9, 218.9, 195.9, 183.9, 170.8, 145.9, 121.9, 116.9. Anal. calcd. for C₂₄H₂₁N₃O₇S: C, 58.17; H, 4.27; N, 8.48. Found: C, 57.48; H, 4.29; N, 8.53.

### EXAMPLE 4: Preparation of (S)-2-[3(S)-benzyl-3-(2-nitrobenzenesulfonyl)amino-2-oxoazetidin-1-yl]propanoic acid (compound of formula (Va): R¹ = CH₂Ph; R²=methyl, R³ = R⁴ = H; R⁹ = 2-nitrobenzenesulfonyl, R¹⁰ = OH; 3S, 1'S configuration)

The process of example 1 was followed, starting from methyl (S)-α-benzyl-serinate (2.1 g, 10 mmol), and (S)-2-(O-tert-butyldimethylsilyloxy)-1-methyl-ethylamine (1.89 g, 10 mmol). Overall yield: 1.56 g (34%); oil.

[α]²⁰_{D} = +95.0 (c = 0.7, MeOH). IR (cm⁻¹, KBr): 1760, 1715 (C=O), 1540 (NO₂), 1360, 1170 (SO₂). ¹H NMR (500 MHz, CD₃OD): δ 8.05-7.66 (m, 4H), 7.27-7.20 (m, 5H), 4.22 (q, 1 H, J = 6.9 Hz), 3.60 (d, 1 H, J = 5.3 Hz), 3.58 (d, 1 H, J = 5.3 Hz), 3.22 (s, 2H), 1.32 (d, 3H, J = 7.0 Hz). ¹³C NMR (75 MHz, CD₃OD): δ 174.6, 168.4, 149.0, 136.3, 135.7, 134.9, 133.7, 131.6, 131.5, 129.5, 128.4, 125.9, 70.5, 51.9, 51.1, 41.0, 15.5. MS m/z (Ion Source Type:
ESI, negative polarity): MS-1: 432.2; MS2(432.2): 385.0, 270.9, 229.9, 201.8, 200.8, 185.9, 137.9; MS3(385.0): 357.0, 283.8, 248.9, 228.8, 200.8, 197.7, 185.8, 172.7, 171.8, 156.8; MS4(200.8): 136.8. Anal. calcd. for C₁₉H₁₉N₃O₇S: C, 52.65; H, 4.42; N, 9.69. Found: C, 52.28; H, 5.21; N, 8.98.

### EXAMPLE 5: Preparation of (R)-2-[3(S)-benzyl-3-(2-nitrobenzenesulfonyl)amino-2-oxoazetidin-1-yl]propanoic acid (compound of formula (Va): R¹ = CH₂Ph; R² = methyl, R³ = R⁴= H; R⁹ = 2-nitrobenzenesulfonyl, R¹⁰ = OH; 3S, 1'R configuration)

The process of example 1 was followed, starting from methyl (S)-α-benzylserinate (2.1 g, 10 mmol), and (R)-2-(O-tert-butyldimethylsilyloxy)-1-methylethylamine (1.89 g, 10 mmol). Overall yield: 2.12 g (46%); oil.

[α]²⁰_{D} = +20.0 (c = 0.7, MeOH). IR (cm⁻¹, KBr): 1750 (C=O), 1715 (C=O), 1540 (NO₂), 1360 (SO₂), 1170 (SO₂).¹H NMR (500 MHz, CD₃OD): δ 8.24-7.76 (m, 4H), 7.32-7.22 (m, 5H), 4.04 (q, 1 H, J = 7.2 Hz), 3.75 (d, 1 H, J = 6.1 Hz), 3.45 (d, 1 H, J = 6.1 Hz), 3.20 (d, 1 H, J = 13.5 Hz), 3.15 (d, 1 H, J = 13.5 Hz), 0.73 (d, 3H, J = 7.5 Hz). ¹³C NMR (75 MHz, CD₃OD): δ 175.5, 167.6, 149.2, 136.4, 135.6, 135.1, 133.8, 131.9, 131.7, 129.5, 128.5, 126.0, 70.6, 51.2, 49.8, 40.6, 15.3. MS m/z (Ion Source Type: ESI, negative polarity): MS-1: 432.2; MS2(432.2): 201.8, 200.8, 185.9, 137.9, 137.0; MS3(200.8): 270.8, 136.8. Anal. calcd. for C₁₉H₁₉N₃O₇S: C, 52.65; H, 4.42; N, 9.69. Found: C, 52.33; H, 5.02; N, 8.65.

### EXAMPLE 6: Preparation of (S)-2-[3-(2-nitrobenzenesulfonyl)amino-3-(3-trifluoromethylbenzyl)-2-oxoazetidin-1-yl]acetic acid (compound of formula (Va): R¹ = 3-trifluoromethylbenzyl; R² = R³ = R⁴ = H; R⁹ = 2-nitrobenzen-sulfonyl, R¹⁰ = OH; 3S configuration)

The process of example 1 was followed, starting from methyl (S)-α-(3-trifluoromethyl-benzyl)serinate (2.8 g, 10 mmol), and 2-(O-tert-butyldimethylsilyloxy)-ethylamine (1.75 g, 10 mmol). Overall yield: 2.12 g (44%); oil.

[α]_{D}²⁷=+ 36.1 (c = 1.2, MeOH); IR (cm⁻¹, KBr): 2922, 2860, 1742 (C=O), 1702 (C=O), 1650, 1540 (NO₂), 1464, 1342 (SO₂), 1174 (SO₂), 1134, 822.¹H-NMR (500MHz, CDCl₃, δ): 8.17 (d, 1 H, J= 7.4 Hz, H₃-arom), 7.89 (d, 1 H, J= 7.5 Hz, H₆-arom), 7.77-7.75 (m, 2H, arom), 7.28 (s, 4H, arom), 5.94 (s, 1 H, SO₂NH), 4.15 (d, 1 H, J= 18.3 Hz, NCHCO), 3.94 (d, 1 H, J= 18.3 Hz, NCHCO), 3.90 (d, 1 H, J= 3.9 Hz, CH₂NCO), 3.72 (d, 1 H, J= 3.9 Hz,

CH₂NCO), 3.24 (s, 2H, CH₂Ph). ¹³C NMR (75 MHz, CD₃OD): δ 170.6, 168.2, 148.7, 135.1, 134.8, 134.5, 134.2, 133.8, 133.5, 130.8, 129.8, 127.9, 126.5, 125.7, 124.7, 71.5, 54.4, 43.2, 40.7. MS m/z (Ion Source Type: ESI, negative polarity): MS-1: 486.2; MS2(486.2): 338.9, 283.9, 239.9, 200.9; MS3(239.9): 224.9, 182.8. Anal. calcd. for C₁₉H₁₆F₃N₃O₇S: C, 46.82; H, 3.31; N, 8.62. Found: C, 47.00; H, 2.99; N, 8.71.

### EXAMPLE 7: Preparation of (S)-2-[3-methyl-3-(2-nitrobenzenesulfonyl)amino)-2-oxoazetidin-1-yl]acetic acid (compound of formula (Va): R¹ = methyl; R² = R³ = R⁴ = H; R⁹ = 2-nitrobenzenesulfonyl, R¹⁰ = OH; 3S configuration)

The process of example 1 was followed, starting from methyl (S)-methyl-serinate (1.3 g, 10 mmol), and 2-(O-tert-butyldimethylsilyloxy)ethylamine (1.75 g, 10 mmol). Overall yield: 1.88 g (55%); oil.

[α]_{D}²⁵ = + 25.4 (c= 1.0, MeOH); IR (cm⁻¹, KBr): 2934, 2860, 1748 (C=O), 1708 (C=O), 1546 (NO₂), 1458 (SO₂), 1168 (SO₂), 822. ¹H-NMR (500MHz, CD₃OD, δ): 8.21-8.18 (m, 1 H, H₃-arom), 7.86-7.83 (m, 1 H, H₆-arom), 7.77-7.75 (m, 2H, arom), 4.13 (d, 1 H, J= 18.3 Hz, NCH₂CO₂H), 3.87 (d, 1 H, J= 18.3 Hz, NCH₂CO₂H), 3.76 (d, 1 H, J= 5.5Hz, CH₂NCO), 3.43 (d, 1 H, J= 5.5Hz, CH₂NCO), 1.59 (s, 3H, Me). ¹³C NMR (75 MHz, CD₃OD): δ 170.8, 169.9, 148.9, 135.9, 134.8, 133.3, 131.3, 125.6, 67.7, 56.4, 43.2, 20.9. MS m/z (Ion Source Type: ESI, negative polarity): MS-2: 341; MS2(341): 283(58), 239(102), 207(134), 169(172), 139(202). Anal. calcd. for C₁₂H₁₃N₃O₇S: C, 41.98; H, 3.82; N, 12.24. Found: C, 42.40; H, 4.30; N, 11.93.

### EXAMPLE 8: Preparation of (R)-3-methyl-2-[13(R)-methyl-(2-nitrobenzenesulfonyl)amino-2-oxoazetidin-1-yl]butanoic acid (compound of formula (Va): R¹ = methyl: R² = isopropyl; R³ = R⁴ = H; R⁹ = 2-nitrobenzenesulfonyl, R¹⁰ = OH; 1'R configuration)

The process of example 1 was followed, starting from methyl (R)-methyl-serinate (1.3 g, 10 mmol), and (R)-2-(O-tert-butyldimethylsilyloxy)-1-isopropylethylamine (2.17 g, 10 mmol). Overall yield: 1.54 g (40%); oil.

[α]_{D}²⁵ = + 22.2 (c = 1.35, MeOH). IR (cm⁻¹, KBr): 3436 (NH), 1755, 1690 (C=O), 1538 (NO₂). ¹H NMR (500 MHz, CD₃OD): δ 8.20-7.81 (m, 4H), 3.96 (d, 1 H, J = 6.0 Hz), 3.80 (d, 1 H, J = 8.0 Hz), 3.37 (d, 1 H, J = 6.0 Hz), 2.18-2.10 (m, 1 H), 1.55 (s, 3H), 0.97 (d, 3H, J = 6.5 Hz), 0.91 (d, 3H, J = 6.5 Hz). ¹³C NMR (75 MHz, CD₃OD): δ 174.8 (b), 168.68, 147.87, 134.90, 133.61,

132.46, 130.56, 124.52, 65.08, 63.77 (b), 54.41, 29.14, 19.99, 18.69, 18.44. MS m/z (Ion Source Type: ESI, negative polarity): MS-1: 383.9; MS2(383.9): 337.0, 292.9, 197.8, 194.8, 185.7, 137.8; MS3(337.0): 292.9, 236.9, 197.8, 153.0. Anal. calcd. for C₁₅H₁₉N₃O₇S: C, 46.75; H, 4.97; N, 10.90. Found: C, 46.98; H, 4.64; N, 10.52.

### EXAMPLE 9: Preparation of methyl (S)-2-methyl-2-[3-benzyl-3-(2-nitrobenzenesulfonyl)amino-2-oxoazetidin-1-yl]propanoate (compound of formula (Va): R¹ = CH₂Ph: R² = R³ =methyl: R⁴ = H; R⁹ = 2-nitrobenzenesulfonyl, R¹⁰ = methoxy: 3S configuration)

o-Nosyl chloride (4.42 g, 20 mmol) was added to a stirred suspension of methyl (S)-α-benzylserinate (2.10 g, 10 mmol) and KHCO₃ (5.0 g, 50 mmol) in acetonitrile (200 mL) and the mixture was stirred at reflux for 16h. Then, methyl α-aminoisobutirate (1.75 g, 15 mmol) in MeCN (50 mL) was added at r.t., and the mixture was stirred at the same temperature for 20h. The reaction mixture was quenched with aqueous NaHCO₃, extracted with EtOAc, and the organic layer was dried (Na₂SO₄) and evaporated to afford methyl (S)-2-benzyl-3-[2-(methoxycarbonylisopropyl)amino]-2-(o-nitrobenzenesulfonyl)aminopropanoate, which was purified by column chromatography (Hex/EtOAc 2:1). To a solution of this intermediate product (5.92 g, 12.5 mmol) in dry THF (150 mL) cooled to 0°C was added LHMDS (1 M sol. in THF) (32 mL, 32 mmol) and the mixture was stirred at the same temperature for 1 hour. The solution was quenched with aqueous NaHCO₃ (150 mL) and extracted with EtOAc (3 x 15 mL). The combined organic fractions were washed with aqueous saturated NaCl (100 mL), dried over MgSO₄, filtered, and evaporated in vacuo to afford the title compound, which was purified by column chromatography (silica-gel 60, eluent Hex/EtOAc 3:1). Yield: 0.325 g (70%); oil.

[α]_{D}²⁵ = - 53.6 (c = 0.1, CH₂Cl₂). IR (cm⁻¹, KBr): 1746, 1713 (C=O). ¹H NMR (CDCl₃, 500 MHz): δ 7.28-8.19 (m, 9H), 3.74 (d, 1 H, J = 5.4 Hz), 3.71 (s, 3H), 3.5 (d, 1 H, J = 5.4 Hz), 3.16 (s, 2H), 1.43 (s, 3H), 1.39 (s, 3H). ¹³CNMR (75 MHz, CDCl₃): δ 172.9, 165.1, 147.3, 135.3, 133.5, 132.9, 131.0, 130.1, 128.7, 127.6, 125.2, 68.1, 58.9, 52.6, 51.2, 40.4, 23.6, 23.5. MS m/z (Ion Source Type: ESI, positive polarity): MS: 460.0, MS2 (460.0): 434.1, 435.0, 247.1, MS3 (434.0): 248.1, 247.1, 147.0, 146.0, 131.1, 130.1, MS4 (247.1): 189.0, 147.1, 145.9, 130.0. Anal. calcd. for C₂₁H₂₃N₃O₇S: C, 54.65, H, 5.02, N, 9.11. Found: C, 54.06, H, 5.25, N, 8.07.

### EXAMPLE 10: Preparation of methyl (S)-1-[3-benzyl-(2-nitrobenzenesulfonyl)amino-2-oxoazetidin-1-yl]cyclopentanecarboxylate (compound of formula (Va): R¹ = CH₂Ph: R² = R³ = together form a cyclopentyl ring: R⁴= H; R⁹= 2-nitrobenzenesulfonyl, R¹⁰ = Methoxy: 3S configuration)

The process of example 9 was followed, starting from methyl (S)-2-benzyl-serinate (2.10 g, 10 mmol) and methyl 1-aminocyclopentanecarboxylate (2.145 g, 15 mmol). Yield: 2.50 g (51 %); yellow oil.

[α]_{D}²⁵ = + 45.5 (c = 1.7, CH₂Cl₂). IR (cm⁻¹, KBr): 3352, 2957 (NH), 1767, 1759 (C=O). ¹H NMR (500 MHz, CDCl₃): δ 8.18 (d, 1 H, J = 7.7Hz), 7.90 (d, 1 H, J = 7.7Hz), 7.76 (m, 4H), 7.31-7.29 (m, 5H), 5.94 (br s, 1 H), 3.74-3.73 (m, 4H), 3.54 (d, 1 H, J= 5.3Hz), 3.22 (s, 2H), 2.35 (m, 1 H), 2.16 (m, 1 H), 2.02 (m, 2H), 1.77 (m, 2H), 1.64 (m, 1 H), 1.54 (m, 1 H). ¹³C NMR (75 MHz, CDCl₃): δ 172.9, 165.8, 147.4, 135.5, 133.8, 133.4, 132.9, 130.9, 130.0, 128.8, 127.7, 125.2, 68.9, 68.0, 52.6, 51.8, 40.5, 35.1, 34.9, 23.7. MS m/z (Ion Source Type: ESI, negative polarity): 288 (10), 219 (13), 133 (12), 120 (15), 97 (12), 92 (25), 91 (100), 71 (11), 67 (24), 57 (15). HRMS (m/z) 487.1420; C₂₃H₂₅N₃O₇S requires 487.1413. 10

### EXAMPLE 11: Preparation of methyl (S)-2-methyl-2-[3-(2-nitrobenzenesulfonyl)amino-3-(2,3,4,5,6-pentafluorobenzyl)-2-oxoazetidin-1-yl]propanoate (compound of formula (Va): R¹= CH₂C₆F₅: R² = R³ = methyl; R⁴ = H; R⁹ = 2-nitrobenzenesulfonyl, R¹⁰ = methoxy: 3S configuration)

The process of example 9 was followed, starting from methyl (S)-2-(2,3,4,5,6-pentafluorobenzyl)serinate (0.284 g, 0.96 mmol), o-NsCl (0.422 g, 1.9 mmol), and H-AibOMe (2 mmol, 0.22 g). Yield: 209 mg (40%).

Mp = 138-139 °C; [α]_{D}²⁵ = +72.1 (c = 1, CH₂Cl₂). IR (cm⁻¹, KBr): 1764, 1748 (C=O), 1102 (C-F), ¹H NMR (500 MHz, CDCl₃): δ 8.14 (m, 1 H), 7.92 (m, 1 H), 7.74 (m, 2H), 6.13 (br s, 1 H), 3.84 (d, 1 H, J = 5.7 Hz), 3.72 (s, 3H), 3.53 (d, 1 H, J = 5.7 Hz), 3.32 (s, 2H), 1.64 (s, 3H), 1.49 (s, 3H). ¹³C NMR (75 MHz, CDCl₃): δ 173.1, 165.9, 147.6, 135.4, 133.5, 132.9, 131.3, 125.3, 68.1, 59.0, 53.4, 52.6, 43.3, 29.7, 24.5, 23.9, 23.8, 22.8. MS (70 eV, EI): m/z: 378(32); 305(10); 298(10); 278(13); 267(15); 264(20); 238(15).

### EXAMPLE 12: Preparation of methyl (S)-2-methyl-2-[3-allyl-3-(2-nitrobenzenesulfonyl)amino-2-oxoazetidin-1-yl]propanoate (compound of formula (Va): R¹ = allyl; R² = R³=methyl; R⁴ = H; R⁹ = 2-nitrobenzenesulfonyl, R¹⁰ = methoxy; 3S configuration)

The process of example 9 was followed, starting from methyl (S)-α-allylserinate (1.59 g, 10 mmol), methyl α-aminoisobutirate (1.75 g, 15 mmol). Yield: 3.16 g (77%); colorless oil.

[α]_{D}²⁵ = +107.1 (c = 1.16, CH₂Cl₂). IR (cm⁻¹, KBr): 3337 (NH), 3100 (C=C), 1760 (C=O), 1541 (NO₂), 1350. ¹H NMR (500 MHz, CDCl₃): δ 8.15-7.71 (m, 5H), 5.93 (s, 1 H), 5.84-5.75 (m, 1 H), 5.24-5.19 (m, 2H), 3.82 (d, 1 H, J = 5.2 Hz), 3.74 (s, 3H), 3.41 (d, 1 H, J = 5.2 Hz), 1.68 (s, 3H), 1.52 (s, 3H). ¹³C NMR (75 MHz, CDCl₃): δ 173.3, 165.6, 147.8, 135.6, 133.6, 133.1, 131.4, 130.6, 125.4, 121.4, 67.0, 59.2, 52.8, 52.5, 39.8, 24.2, 23.8. MS m/z (Ion Source Type: ESI positive polarity): MS+1: 412.2; MS2(412.2): 320.2, 300.1, 384.1; MS3(320.2): 208.0. MS4(208.0): 100.0, 163.0, 132.9, 135.0, 193.0. Anal. calcd. for C₁₇H₂₁N₃O₇: C, 49.63; H, 5.14; N, 10.21; Found: C, 49.80; H, 4.79; N, 10.27.

### EXAMPLE 13: Preparation of methyl (S)-2-methyl-2-[3-(formylmethyl)-3-(2-nitrobenzenesulfonyl)amino-2-oxoazetidin-1-yl]propanoate (compound of formula (Va): R¹ = CH₂CHO; R² = R³ = methyl; R⁴ = H; R⁹ = 2-nitrobenzenesulfonyl, R¹⁰ = methoxy; 3S configuration)

To a solution of K₂OsO₂(OH) (4 mg, 0.01 mmol) in ^{t}BuOH/H₂O (1/1, 30 mL) cooled to 0°C were added DABCO (0.112 g, 1 mmol), K₃FeCN₆ (0.989 g, 3 mmol), K₂CO₃ (0.415 g, 3 mmol) and (3S)-3-allyl-1-[1-(methoxycarbonyl)-isopropyl]-3-(2-nitrobenzenesulfonamido)-azetidin-2-one (0.42 g, 1 mmol; prepared according to example 12). The resulting suspension was stirred at room temperature overnight and then EtOAc (15 mL) was added and the organic layer was washed with H₂O (2 mL). The organic phase was dried over MgSO₄, and the solvent was evaporated under reduced pressure giving an inseparable mixture of two epimers of (3S)-3-(2,3-dihydroxypropyl)-1-[1-(methoxycarbonyl)-isopropyl]-3-(2-nitrobenzenesulfonamido)-azetidin-2-one, which was purified by flash chromatography (hexane/EtOAc 1:1). Yield: 0.325 g (71 %); colorless oil.

IR (cm⁻¹, KBr): 3280 (NH), 2923 (Ar), 1760 (C=O), 1542, 1354, 1165. ¹H NMR (500 MHz, CDCl₃): (two stereoisomers) δ 8.14-7.68 (m, 4H), 4.17-3.30 (m, 10H), 3.73 (s, 3H), 3.72 (s, 3H), 2.18-1.95 (m, 4H), 1.69 (s, 3H), 1.64 (s, 3H), 1.49 (s, 3H), 1.46 (s, 3H). ¹³C NMR (75 MHz, CDCl₃): δ (two stereoisomers) 176.5, 174.2, 173.3, 167.4, 167.2, 147.6, 136.0, 135.8, 133.3, 133.3, 133.0, 132.6, 131.0, 130.8, 130.6, 125.3, 125.0, 124.9, 70.1, 69.4, 68.8, 67.7, 67.2, 66.4, 66.2, 63.9, 63.4, 59.1, 59.0, 53.4, 52.8, 52.7, 52.2, 48.9, 37.8, 37.5, 34.5, 29.7, 25.8, 24.9, 23.8, 23.7. MS m/z (Ion Source Type: ESI positive polarity): MS+1:446.1; MS2(446.1): 386.0, 130.0; MS3(386.0): 156.0, 138.0, 185.9. MS4(156.0): 138.0, 94.1.

This diol product (0.79 mmol, 0.325 g) was then dissolved in CH₂Cl₂ (5mL) at 0°C and NalO₄ supported on silica (3.2 g; prepared according to J. Org. Chem. 1997, vol. 62, pp. 2622-2624) was added. The mixture was stirred at room temperature for 2h, and then filtered over celite. Evaporation of the filtrate under reduced pressure gave the aldehyde product. Yield: 0.270 g (90%); colorless oil.

[α]_{D}²⁵ = +42.6 (c = 0.85, CH₂Cl₂). IR (cm⁻¹, KBr): 3330 (NH), 2949 (Ar), 1737 (C=O), 1541, 1349, 1165. ¹H NMR (500 MHz, CDCl₃): δ 9.76 (s, 1 H), 8.18-7.72 (m, 4H), 6.36 (s, 1 H), 3.78 (d, 1 H, J = 6 Hz), 3.72 (s, 3H), 3.44 (d, 1 H, J = 6 Hz), 3.11 (d, 1 H, J = 18 Hz), 3.06 (d, 1 H, J = 18 Hz), 1.67 (s, 3H), 1.48 (s, 3H). ¹³C NMR (75 MHz, CDCl₃): δ 173.1, 164.2, 147.6, 135.1, 133.8, 133.0, 130.9, 125.3, 64.8, 59.3, 52.6, 46.9, 29.6, 23.8. MS m/z (Ion Source Type: ESI positive polarity): MS+1: 414.0; MS2(414.0): 168.0, 386.0, 368.0, 211.9, 130.0; MS3(386.0): 368.0, 139.0, 199.0. MS4(368.0): 182.0, 181.0, 153.0, 123.0, 121.0. Anal. calcd. for C₁₆H₁₉N₃O₈S: C, 46.49; H, 4.63; N, 10.16. Found: C, 46.29; H, 5.11; N, 8.70.

### EXAMPLE 14: Preparation of methyl (S)-2-methyl-2-[3-(2-nitrobenzenesulfonyl)amino-3-propargyl-2-oxoazetidin-1-yl]propanoate (compound of formula (Va): R¹ = propargyl; R² = R³ = methyl; R⁴ = H; R⁹ = 2-nitrobenzenesulfonyl, R¹⁰ = methoxy: 3S configuration)

To a solution of (3S)-3-(formylmethyl)-1-[1-(methoxycarbonyl)-isopropyl]-3-(2-nitrobenzenesulfonamino)-azetidin-2-one (0.544 g, 1.32 mmol; prepared according to example 13) in dry MeOH (15 mL) cooled at 0°C, dimethyl (1-diazo-2-oxopropyl)phosphonate (0.380 g, 1.98 mmol; prepared according to Synlett 1996, vol.6, pp. 521-522) was added followed by K₂CO₃ (0.365 g, 2.64 mmol). The reaction mixture was stirred at 0°C for 1 h, and then at room temperature for 4h. After concentrating the mixture under reduced pressure, EtOAc (15 mL) was added, the organic extract was washed with saturated aqueous NaHCO₃ (3x10 mL) and back-extracted with EtOAc (20 mL). The organic layers were combined and washed with brine (15 mL), dried (MgSO₄), filtered, and evaporated. The crude oil was purified by flash chromatography (60 mesh silica gel, hexane/EtOAc 2:1). Yield: 0.450 g (83%); colorless oil.

[α]_{D}²⁵ = - 44.6 (c = 1.32, CH₂Cl₂). IR (cm⁻¹, KBr): 3285 (NH), 2955, 2070 (C=C), 1760 (C=O), 1538, 1353, 1164. ¹H NMR (500 MHz, CDCl₃): δ 8.18-7.72 (m, 4H), 6.22 (s, 1 H), 3.80 (d, 1 H, J = 5.5 Hz), 3.80 (s, 3H), 3.52 (d, 1 H, J = 5.5 Hz), 2.79 (d, 2H, J = 2.5 Hz), 2.10 (dd, 1 H, J = 2.5 Hz), 1.67 (s, 3H), 1.54 (s, 3H). ¹³C NMR (75 MHz, CDCl₃): δ 173.0, 164.1, 147.6, 135.4, 133.7, 133.1, 130.9, 125.4, 76.6, 72.9, 66.3, 59.2, 52.7, 52.4, 25.1, 23.9, 23.9. MS m/z (Ion Source Type: ESI positive polarity): MS+1: 410.2; MS2(410.2): 382.1, 195.1 MS3(382.1): 196.0, 195.0, 135.1; MS4(195.0) 167.0, 137.1, 135.1, 95.2. Anal. calcd. for C₁₇H₁₉N₃O₇S:C, 49.87; H, 4.68; N, 10.26. Found: C, 49.55; H, 4.50; N, 9.89.

### EXAMPLE 15: Preparation of methyl (S)-2-methyl-2-[3-(2-nitrobenzenesulfonyl)amino-3-([1-(2,3,4,6-tetra-O-benzyl-α-D-mannosyl)-1,2,3-triazol-4-yl]methyl)-2-oxoazetidin-1-yl]propanoate (compound of formula (Va): R¹ = [1-(2,3,4,6-tetra-O-benzyl-α-D-mannosyl)-1,2,3-triazol-4-yl]methyl: R² = R³=methyl; R⁴ = H; R⁹ = 2-nitrobenzenesulfonyl, R¹⁰ = methoxy: 3S configuration)

To a solution of (3R)-1-[1-(methoxycarbonyl)-isopropyl]-3-(2-nitro-benzene-sulfonamino)-3-propargyl-azetidin-2-one (0.04 g, 0.10 mmol; prepared according to example 14) and 2,3,4,6-tetra-O-benzyl-α-D-mannosyl azide (0.06 g, 0.10 mmol) in a 1:1:1 mixture of THF (2 mL), H₂O (2 mL) and ^{t}BuOH (2 mL) was added copper(II)sulfate (0.32 mL, 0.06 mmol of freshly prepared 0.2 M solution in H₂O), followed by sodium ascorbate (0.13 mL, 0.13 mmol of freshly prepared 1 M solution in H₂O). The reaction mixture was vigorously stirred overnight at room temperature. After completion (monitored by TLC) the reaction mixture was concentrated in vacuo and the residue was purified by flash column chromatography (hexane/EtOAc 1:1). Yield: 0.06 g (60 %); colorless oil.

[α]_{D}²⁵ = +0.6 (c = 1, CH₂Cl₂). IR (cm⁻¹, KBr): 3320, 2923, 2854 (NH), 1761 (C=O), 1540 (NO₂), 1453, 1360, 1115 (triazole), 769 (CH). ¹H NMR (500 MHz, CDCl₃): δ 8.17-7.59 (m, 5H), 7.70 (s, 1 H), 7.34-7.12 (m, 20H), 6.42 (s, 1 H), 5.86 (d, 1 H, J = 3.6 Hz), 4.76 (dd, 1 H, J = 3.2 Hz), 4.72-4.58 (m, 9H), 4.05 (dd, 1 H, J = 10.3 Hz), 3.96 (dd, 1 H, J = 8.0 Hz), 3.78 (d, 1 H, J = 5.5 Hz), 3.72-3.62 (m, 3H), 3.70 (s, 3H), 3.61 (d, 1 H, J = 5.5 Hz), 3.29 (d, 2H, J = 10.2 Hz), 1.61 (s, 3H), 1.46 (s, 3H). ¹³C NMR (75 MHz, CDCl₃): δ 173.2, 165.3,

147.6, 141.4, 138.3, 138.3, 138.2, 137.8, 135.6, 133.6, 133.1, 131.3, 128.7, 128.6, 128.5, 128.2, 128.1, 128.1, 127.9, 127.8, 125.4, 123.3, 85.2, 78.4, 77.4, 75.2, 74.4, 74.3, 74.2, 73.6, 72.8, 68.9, 67.4, 59.3, 52.9, 52.6, 31.1, 29.9, 24.1. MS m/z (Ion Source Type: ESI positive polarity): MS+1: 975.4 MS2(975.4): 634.2, 633.2, 544.1, 543.2, 453.2; MS3(543.2): 515.0, 414.1, 329.2, 328.2, 183.0; MS4(328.2): 300.2, 228.0, 183.0. Anal. calcd. for C₅₁H₅₄N₆O₁₂S: C, 62.82; H, 5.58; N, 8.62. Found: C, 62.87; H, 6.03; N, 8.44.

### EXAMPLE 16: Preparation of (S)-2-methyl-2-[3-(2-nitrobenzenesulfonyl)amino)-3-benzyl-2-oxoazetidin-1-yl]propanoic acid (compound of formula (Va): R¹ = CH₂Ph: R² = R³ = methyl; R⁴ = H; R⁹ = 2-nitrobenzenesulfonyl, R¹⁰ = OH; 3S configuration)

LiOH·H₂O (3.78 g, 90 mmol) was added to a suspension of methyl (S)-2-methyl-2-[3-benzyl-3-(2-nitrobenzenesulfonyl)amino-2-oxoazetidin-1-yl]propanoate (4.15 g, 9 mmol; prepared according to example 9) in THF/H₂O (100/12 mL) and the mixture was stirred vigorously at the same temperature for 2h 30min. Dichloromethane (50 mL) was added, and the mixture was extracted with aqueous saturated NaHCO₃ (3 x 50 mL). The aqueous solutions were combined, acidified with 0.1 M HCl until acidic pH, and extracted with CH₂Cl₂ (3 x 50 mL). The organic solution was dried (MgSO₄) and evaporated to afford pure product. Yield: 100%; Oil.

[α]_{D}²⁵ = +2.4 (c= 1.1, MeOH); ¹H-NMR (500MHz, CDCl₃, δ): 8.16 (d, 1 H, J= 8.8 Hz, arom), 7.89 (d, 1 H, J= 8.8 Hz, arom), 7.75-7.71 (m, 2H, arom), 7.27 (s, 5H, arom), 6.02 (bs, 1 H, SO₂NH), 3.79 (d, 1 H, J= 5.1 Hz, CH₂NCO), 3.54 (d, 1 H, J= 5.1 Hz, CH₂NCO), 3.17 (s, 2H, CCH₂Ph), 1.42 (s, 6H, (CH₃)₂CO₂H).

### EXAMPLE 17: Preparation of methyl (S)-2-methyl-2-[3-(2-nitrobenzenesulfonyl)amino)-3-propargyl-2-oxoazetidin-1-yl]propanoate (compound of formula (Va): R¹ = propargyl; R² = R³=methyl; R⁴ = H; R⁹ = 2-nitrobenzenesulfonyl, R¹⁰ = OH; 3S configuration)

The process of example 16 was followed, starting from (S)-2-methyl-2-[3-(2-nitrobenzenesulfonyl)amino-3-propargyl-2-oxoazetidin-1-yl]propanoate (1.04 g, 5 mmol; prepared according to example 14). Yield: 95%; Oil.

[α]_{D}²⁵ = +7.5 (c= 1.0, CH₂Cl₂); IR (cm⁻¹, KBr): 3340 (NH), 2940 (C≡C), 1735, 1712 (C=O), 1450, 1353, 911, 728. ¹H-NMR (500MHz, CDCl₃, δ): 8.18-8.17 (m, 1 H, arom), 7.92-7.90 (m, 1 H, arom), 7.76-7.73 (m, 2H, arom), 6.49 (s, 1 H, SO₂NH), 3.82 (d, 1 H, J= 5.6 Hz, CH₂NCO), 3.56 (d, 1 H, J= 5.6 Hz, CH₂NCO), 2.82 (d, 2H, J= 2.4 Hz, CH₂C=), 2.09 (bs, 1 H, CH), 1.67 (s, 3H, CH₃), 1.56 (s, 3H, CH₃). ¹³C NMR (75 MHz, CDCl₃): δ 174.2, 165.2, 147.8, 134.9, 133.7, 132.3, 130.3, 124.6, 77.0, 72.0, 65.9, 59.0, 50.4, 23.8, 22.9, 22.8. MS m/z (Ion Source Type: ESI positive polarity): MS-1: 394.0; MS2(394.0): 191.8 (202.2), 200.7(193.3); 346.9 (47.1) MS3(191.8): 147.8(44), 107.9(83.9). Anal. calcd. for C₁₆H₁₇N₃O₇S: C, 48.36; H, 4.82; N, 10.57. Found: C, 49.00; H, 4.90; N, 10.90.

### EXAMPLE 18: Preparation of 2-NO₂C₆H₄-SO₂-(beta-Lactam)-Arg(Pbf)-OBn (compound of formula (IV): R7 = 2-nitrobenzenesulfonyl; Y = -(beta-Lactam)-Arg(R⁵)-; R¹ = CH₂Ph; R² = R³ = methyl; R⁴ = H; R⁵ = Pbf; R⁸ = OBn; 3S configuration).

A solution of (S)-2-methyl-2-[3-(2-nitrobenzenesulfonyl)amino-3-benzyl-2-oxoazetidin-1-yl]propanoic acid (1.94 g, 4.3 mmol; prepared according to example 16) in anhydrous CH₂Cl₂ (60 mL) was cooled at -10°C and a solution of H-Arg(Pbf)-OBn (2.32 g, 5.6 mmol) in CH₂Cl₂ (10 mL) was added. Then, EEDQ (2.13 g, 8.6 mmol) was added and the mixture was stirred for 16h while slowly warming to room temperature. The reaction mixture was washed consecutively with 1 M HCl and saturated NaHCO₃ and the organic layer was dried over MgSO₄ an evaporated under reduced pressure. The crude was purified by column chromatography (eluent: Hex/EtOAc 1/50). Yield: 65%. Oil.

[α]_{D}²⁵ = +26.4 (c= 1.0, CH₂Cl₂); IR (cm⁻¹, KBr): 3375 (NH); 1748 (C=O); 1613 (C=O); 1543, 1457, 1349 (HN-CO); HPLC-MS, MeOH/COOH m/z (Ion Source Type: ESI, positive polarity): MS1=946.10 (M*); MS2(946.5); 676.4(270.1); MS3(676.4): 648.4(28.0); 619.4(57); 316.3(360.1). ¹H-NMR (δ, ppm, CDCl₃): 8.28 (m, 1 H, H₃-Ns); 7.90 (m, 1 H, H₆-Ns); 7.77-7.71 (m, 2H, H₄-Ns, H₅-Ns); 7.44 (d, 1 H, J=7.9Hz, NHaArg); 7.37-7.26 (m, 10H, Ar); 6.47 (s, 1 H, SO₂NH); 6.05 (bs., 2H, NHωArg); 5.22-5.14 (m, 2H, CO₂CH₂Ph); 4.56 (m, 1 H, CHαArg); 3.58 (d, 1 H, J=5.8Hz, NCH₂C); 3.49 (d, 1 H, J=5.8Hz, NCH₂C); 3.20 (m, 1 H, CH₂Argδ); 3.15 (m, 2H, J₁=13.4, J₂=29.7Hz, CCH₂Ph); 3.10 (overload, 1 H, CH₂Argδ); 2.96 (s, 2H, CH₂Pbf); 2.59 (s, 3H, CH₃Pbf); 2.53 (s, 3H, CH₃Pbf); 2.10 (s, 3H, CH₃Pbf); 1.87 (m, 2H, CH₂βArg); 1.61 (m, 2H, CH₂γArg); 1.47 (s, 6H, 2xCH₃Pbf); 1.45 (s, 3H, CH₃Aib); 0.86 (s, 3H, CH₃Aib). ¹³C-NMR (δ, ppm, CDCl₃): 173.2; 171.6; 165.5; 158.6; 156.0; 147.2; 138.2; 135.3; 134.7; 133.7; 133.2; 133.0; 132.2; 131.2; 130.5; 128.5; 128.3; 127.9; 125.0; 124.5; 117.4; 86.3; 69.0; 67.0; 60.6; 52.4; 51.0; 43.1; 40.7; 40.0; 28.7; 28.5; 26.0; 24.1; 23.0; 19.2; 17.9; 14.1; 12.3. Anal. Calcd. For C₄₆H₅₅N₇O₁₁S₂ (946.0992): C, 58.40; H, 5.86; N, 10.36. Found: C, 56.10; H, 5.90; N, 10.10.

### EXAMPLE 19: Preparation of Cbz-Asp(O^{t}Bu)-(beta-Lactam)-OH (compound of formula (IV): R⁷ = Cbz; Y = -Asp(R⁶)-(beta-Lactam)-; R¹ = R² = R³ = H; R⁴ = (4S)-2,2-dimethyl-1,3-dioxolan-4-yl; R6=O^{t}Bu, R⁸=OH; 3S, 4R configuration).

To a stirred solution of (R)-2,2-dimethyl-1,3-dioxolane-4-carbaldehyde (12.4 g, 33 mmol) in CH₂Cl₂ (150 mL) was added, at 0 °C, 2-(tert-butyldimethyl-silyloxy)ethanamine (14.3 g, 30 mmol) and molecular sieves (4A). The mixture was stirred over 60 minutes at same temperature. Then, it was filtered off and the solvent was evaporated to afford the corresponding imine. This imine was dissolved in CH₂Cl₂ (150mL) and the solution was cooling to -78 °C. Then, Et₃N (8.4 mL, 60 mmol) was added. Finally a solution of benzyloxyacetyl chloride (13.5 g, 33 mmol) in CH₂Cl₂ (150 mL) was added drop-wise at same temperature, and the mixture was stirred overnight allowing it to reach r.t. Then, the solution was washed consecutively with water, 1 M HCl and sat. soln. of NaHCO₃ and it was dried over MgSO₄. The solvent was evaporated in vacuo and the resulting oil was purified by column chromatography (basic silica gel; eluent: Hex/EtOAc 95:5) to give (3R, 4S)-3-(benzyloxy)-1-[2-(tertbutyldimethylsilyl)ethyl]-4(S)-(2,2-dimethyl-1,3-dioxolan-4-yl)azetidin-2-one. Yield: 60 % (7,84g).

To a stirred solution of (3R, 4S)-3-(benzyloxy)-1-[2-(tert-butyldimethylsilyl)-ethyl]-4(S)-(2,2-dimethyl-1,3-dioxolan-4-yl)azetidin-2-one (7.48 g, 18 mmol)

in methanol (180 mL), Pd-C (800 mg) and ammonium formiate (7.2 g, 111 mmol) was added and the mixture was heated to reflux over 2 hour. Then the mixture was filtered through celite and the solution was poured into CH₂Cl₂ (250 mL) and washed with 0.1 N HCl (75 mL). The organic layer was separated and dried over MgSO₄. Solvents were evaporated in vacuo to give (3R, 4R)-1-[2-(tert-butyldimethylsilyloxy)ethyl]-4-[(S)-2,2-dimethyl-1,3-dioxolan-4-yl]-3-hydroxyazetidin-2-one a black solid, which was used without further purification in the next step. Yield: 95% (5.91 g).

A solution of (3R, 4R)-1-[2-(tert-butyldimethylsilyloxy)ethyl]-4-[(S)-2,2-dimethyl-1,3-dioxolan-4-yl]-3-hydroxyazetidin-2-one (5.90 g, 17.1 mmol) in CH₂Cl₂ (200 mL) was cooled at 0 °C, followed by the addition of Et₃N (5 mL, 34.2 mmol). The solution was stirred for 15 minutes and 2-nitrobenzenesulfonyl chloride (4.16 g, 18.81 mmol) was added. The solution was stirred for 4 h at 0°C and then washed with brine. The organic phase was dried over MgSO₄, filtered and concentrated in vacuo. The resulting solid was used in the next step without further purification. Yield: 85 % (7.71 g)

A solution of (2S, 3R)-1-[2-(tert-butyldimethylsilyloxy)ethyl]-4-[(S)-2,2-dimethyl-1,3-dioxolan-4-yl]-4-oxoazetidin-3-yl-2-nitrobenzenesulfonate (7.71 g, 14.5 mmol) in DMF (130 mL) was heated at 80 °C. Then, sodium azide (4.80 g, 72.5 mmol) was added in one portion. The reaction mixture was stirred for 4 h at 80 °C and then cooled to room temperature and treated with water. The product was extracted with several portions of diethyl ether, which were combined and washed several times with brine. Then the organic layer was dried over MgSO₄, filtered, evaporated and the resulting oil was purified by column chromatography (basic silica gel; eluent: (Hex/EtOAc 95:5) to give (3S, 4R)-3-azido-1-[2-(tert-butyldimethylsilyloxy)ethyl]-4-[(S)-2,2-dimethyl-1,3-dioxolan-4-yl]azetidin-2-one. Yield: 70% (3.76 g).

A solution of (3S, 4R)-3-azido-1-[2-(tert-butyldimethylsilyloxy)ethyl]-4-[(S)-2,2-dimethyl-1,3-dioxolan-4-yl]azetidin-2-one (3.76 g, 10.2 mmol) in ethanol (100 mL) was stirred with Lindlar catalyst (400 mg, 5% Pd/CaCO₃) under hydrogen atmosphere by 16 hours. After this time the catalyst was removed by filtration through celite and the residue was dried over MgSO₄, and evaporated in vacuo to give (3S, 4R)-3-amino-1-[2-(tert-butyldimethylsilyloxy]ethyl)-4-[(S)-2,2-dimethyl-1,3-dioxolan-4-yl]azetidin-2-one which was used in the next step without further purification. Yield: 95% (3,32 g).

To a stirred solution of (3S, 4R)-3-amino-1-(2-(tert-butyldimethylsilyloxy)ethyl)-4-[(S)-2,2-dimethyl-1,3-dioxolan-4-yl)azetidin-2-one (3.318 g, 9.6mmol) in dry CH₂Cl₂ (100mL) were added successively Cbz-L-aspartic acid-4-tert-butyl ester 3.318g, 9.6mmol), EDC (2.9g, 15.4mmol), HOBT (2.03g, 13.4mmol) and TEA (2.8mL, 19.2mmol) and the mixture was stirred for 20 h at room temperature. After this time the solution was washed with HCl 0.1 M (2 X 50mL) and saturated solution of NaHCO₃ (2 X 50mL). The organic layer was dried over MgSO₄ and evaporated and the crude was purified by basic flash chromatography (Hex/EtOAc 95:5), yielding Cbz-Asp(O^{t}Bu)-(3S, 4R)-3-amino-1-(2-(tert-butyldimethylsilyloxy)ethyl)-4-[(S)-2,2-dimethyl-1,3-dioxolan-4-yl]azetidin-2-one. Yield: 80 % (5g).

Cbz-Asp(^{t}Bu)-(3S, 4R)-3-amino-1-(2-(tert-butyldimethylsilyloxy)ethyl)-4-[(S)-2,2-dimethyl-1,3-dioxolan-4-yl)azetidin-2-one (5 g, 7.68 mmol) in THF (40ml) was cooled at 0 °C. Then hydrogen fluoride-pyridine complex (12 mL,8.4 mmol) was added and after 30 minutes the ice bath was removed. Stirring is continued while the reaction is monotorized by T.L.C. (Hex./EtOAc 1:5) and additional amounts of hydrogen fluoride-pyridine were added until the reaction was completed. The cold solution is diluted with ether (300ml) and neutralized with saturated sodium hydrogen carbonate solution until no more carbon dioxide is liberated. The organic layer is separated and washed with saturated sodium chloride (50ml), dried over MgSO₄, and concentrated. Toluene was added and the solution was concentrated again to remove traces of pyridine to give (S)-tert-butyl 3-(benzyloxycarbonylamino)-4-((2R, 3S)-2-((S)-2,2-dimethyl-1,3-dioxolan-4-yl)-1-(2-hydroxyethyl)-4-oxoazetidin-3-ylamino)-4-oxobutanoate. The product was used in the next step without further purification. Yield: 90% (3.7g).

BAIB (4.93 g, 15.2 mmol), TEMPO (90 mg, 0.56 mmol) and the (S)-tert-butyl 3-(benzyloxycarbonylamino)-4-((2R, 3S)-2-((S)-2,2-dimethyl-1,3-dioxolan-4-yl)-1-(2-hydroxyethyl)-4-oxoazetidin-3-ylamino)-4-oxobutanoate (3.7 g, 6.9 mmol) were added to a mixture of acetonitrile/water (10:10 mL). The reaction mixture was stirred for 4h. After this time CH₂Cl₂ (40 mL) is added and the compound is extracted several times from the water solution. The organic layer was dried over MgSO₄ and evaporated under reduced pressure. The crude was purified by column chromatography (eluent: Hex/EtOAc 1/12). Yield: 70% (2.65 g).

M.p.: 128-131 [α]_{D}²⁵ = -8.80 (c= 0.5 CH₂Cl₂); IR (cm⁻¹, KBr): 3308, 3034, 2980, 2926, 2854, 1760, 1733, 1700. ¹H-NMR (δ, ppm, CDCl₃) 7.95 (1 H, d, NH[β-lac], J= 6.2Hz); 7.31 (5H, m, arom.); 6.04 (1 H, d, NH-Asp., J= 8.5Hz); 5.09 (2H, m, CH₂-Bn); 4.68 (1 H, m, H^{α}s[β-lac]); 4.56 (1 H, m, H^{α}-Asp); 4.26 (1 H, d, CHH-COOH, J=17.9Hz); 4.16 (1 H, m, Hₛdioxol); 4.07 (1 H, m, CHH-dioxo,); 3.95 (1 H, d, CHH-COOH, J=17.9Hz); 3.75 (1 H, m, CHH-dioxo); 3.63 (1 H, dd, H_{R} [β-lac], J=1.8 J=8.2); 2.86 (1 H, dd, H^{β}-Asp, J=5.5 Hz, J= 16.8 Hz); 2.62 (1 H, dd, H^{β}-Asp, J=4.7 Hz, J= 16.8 Hz ); 1.39 (9H, ^{t}But); 1.34 (3H, CH₃-dioxol.); 1.28 (3H, CH₃-dioxol.). ¹³C-NMR (δ, ppm, CDCl₃): 171.72, 171.63, 170.50, 167.59, 156.50, 136.22, 128.73, 128.42, 128.33, 110.07, 81.96, 75.26, 67.45, 66.77, 61.00, 56.58, 51.83, 43.83, 36.92, 28.17, 26.77, 25.11

### EXAMPLE 20: Preparation of Cbz-Asp(O^{t}Bu)-(beta-Lactam)-OH (compound of formula (IV): R⁷ = Cbz; Y = -Asp(R⁶)-(beta-Lactam)-; R¹ = R² = R³ = H; R⁴ = (4S)-2,2-dimethyl-1,3-dioxolan-4-yl; R⁶= O^{t}Bu, R⁸ = OH; 3R, 4R configuration).

To a stirred solution of (R)-2,2-dimethyl-1 ,3-dioxolane-4-carbaldehyde (12.4 g, 33 mmol) in CH₂Cl₂ (150mL) was added, at 0 °C, 2-(tert-butyldimethyl-silyloxy)ethanamine (14.3 g, 30 mmol) and molecular sieves (4A). The mixture was stirred over 60 minutes at same temperature. Then, it was filtered off and the solvent was evaporated giving the corresponding imine. This imine was dissolved in CH₂Cl₂ (150mL) at room temperature. Then, Et₃N (8.4mL, 60 mmol) was added. Finally, a solution of phthalyglycyl chloride (7.38 g, 33 mmol) in CH₂Cl₂ (150mL) was added drop-wise at the same temperature. The mixture was stirred over night. After this time the reaction was washed with water, HCl (1 M) and sat. sol. of NaHCO₃ and was dried over MgSO₄ . The solvent was evaporated in vacuo and the resulting oil was purified on basic silica gel (Hex./EtOAc 95:5) to give (3R, 4S)-3-benzyloxy-1-(2-(tert-butyldimethylsilyl)ethyl)-4(S)-(2,2-dimethyl-1,3-dioxolan-4-yl)azetidin-2-one. Yield: 70 % (9.97g).

To a stirred solution of (3R, 4S)-3-(benzyloxy)-1-(2-(tert-butyldimethylsilyl)-ethyl)-4-(S)-2,2-dimethyl-1,3-dioxolan-4-yl)azetidin-2-one (9.96 g, 21 mmol) in ethanol (100 mL) the hydrazine (5mL, 100mmol) was added and the solution was vigorously stirred at room temperature for 48 hours. After that time the solution is cooled and filtrated and the solution is evaporated. The precipitate is washed with ether, the combined organic layer was collected and washed with NaHCO₃ dried with MgSO₄ and evaporated in vacuo to give (3R, 4R)-3-amino-1-(2-(tert-butyldimethylsilyloxy)ethyl)-4-((S)-2,2-dimethyl-1,3-dioxolan-4-yl)azetidin-2-one which was used in the next step without further purification. Yield: 75 % (5.42g).

Coupling with Cbz-Asp(O^{t}Bu)-OH was conducted as described in example 19. M.p.: 108-110 [α]_{D}²⁵ = -15.35 (c= 0.5 CH₂Cl₂); IR (cm⁻¹, KBr): 3306 3035 2984 2942 1761 1743 1662 ¹H-NMR (δ, ppm, CDCl₃): ¹H: 7.85 (1 H, d, NH-β-lac, J= 8.13); 7.31 (5H, m, arom.); 6.06 (1 H, d, NH-Asp, J= 876 Hz); 5.20 (1 H, dd, H^{α}_{R}[β -Lac], J=5.07 J= 8.35 Hz) ; 5.08 (2H, m, CH₂-Bn); 4.56 (1 H, m, Hα-Asp); 4.15 (2H, m, Hₛdioxol, CHH-COOH); 3.97 (2H, m, CHH-dioxo, CHH-COOH); 3.83 (1 H, m, H^{β}_{R} [β-lac]); 3.63 (1 H, dd, CHH-dioxo, J=4.43, J=8.94); 2.80 (1 H, dd, Hβ-Asp, J=5.61 Hz, J= 16.85 Hz); 2.66 (1 H, dd, Hβ-Asp, J=5.61 Hz, J= 16.85 Hz); 1.38 (9H, m, ^{t}But, CH₃-dioxol); 1.36 (3H, s, CH₃-dioxol.).1.25 (3H, s, CH₃-dioxol.) ¹³C-NMR (δ, ppm, CDCl₃): 171.62, 170.71, 170.43, 167.43, 156.49, 136.02, 128.60, 128.25, 109.95, 81.89, 75.18, 67.41, 66.19, 60.97, 56.99, 51.64, 43.03, 37.49, 28.04, 26.63, 24.96.

### EXAMPLE 21: Preparation of Cbz-Asp(O^{t}Bu)-(beta-Lactam)-Ara(Pbf)-OBn (compound of formula (III): PG¹ = Cbz; Z = Asp(R⁶)-(beta-Lactam)-Arg(R⁵); R¹ = CH₂Ph; R² = R³ = methyl; R⁴ = H; R⁵ = Pbf; R⁶ = O^{t}Bu; PG² = OBn; 3S configuration)

To a stirred solution of peptide 2-NO₂C₆H₄-SO₂-(beta-Lactam)-Arg(Pbf)-OBn (2.65 g, 2.80 mmol, prepared according to example 18) in MeCN/DMSO (30/2.5 mL), thiophenol (0.57 mL, 5.60 mmol) and K₂CO₃(387 mg, 2.80 mmol) were added. The suspension was stirred at room temperature until the reaction completion (1-3 h, monitored by TLC and ¹H NMR). Then, solid K₂CO₃ (3g) was added, the mixture was stirred during 15 min at room temperature and it was filtered through a pad of celite. The filtrate was evaporated in vacuo to afford a mixture of the intermediate α-amino-β-lactam peptide and 2-thiophenyl-nitrobenzene, which was not separated for the next step. To a cooled (-10°C) solution of the former amine (≈ 2.8 mmol) in anhydrous DMF (30 mL) was added Cbz-Asp-(O^{t}Bu)-OH (0.90 g, 2.8 mmol), KHCO₃ (1.40 g, 14 mmol) and HATU (1.60 g, 4.2 mmol). The mixture was stirred for 16 h while warming slowly to room temperature. The solvent was evaporated at reduced pressure, but avoiding complete dryness. Then, EtOAc (50 mL) was added and the resulting solution was washed consecutively with 0.1 M HCl (2 x 20 mL), saturated NaHCO₃ (2x20 mL) and brine (20 mL). The organic layer was dried (MgSO₄) and evaporated under reduced pressure. The product was purified by column chromatography (silica gel, eluent: EtOAc/Hexane 5/1). Yield: 75 %. Oil.

[α]_{D}²⁵ = -44.0 (c= 0.2, CH₂Cl₂); IR (cm⁻¹, KBr): 3428, 3332 (NH); 2987, 2911 (C-C); 1748, 1667 (C=O); 1549, 1452, 1366, 1258 (NHC=O); 1167. HPLC-MS, MeOH/COOH m/z (Ion Source Type: ESI, positive polarity): MS1=1066.3=M*; MS2(1066.5)=915.6(150.9); 1000.6(85.1); MS3(1000.6)=915.6(85); 835.5(165.1).¹H-NMR (δ, ppm, CDCl₃): 8.18 (bs, 1 H, NHαArg); 7.38-7.25 (m, 15H, Ar); 7.03 (s, 1 H, β-LactamNH); 6.21 (d, 1 H, J=7.2Hz, NHαAsp); 5.95 (bs., 2H, NHωArg); 5.15-5.08 (m, 4H, CO₂CH₂Ph); 4.48 (m, 1H, CHαAsp); 4.27 (m, 1H, CHαArg); 3.50 (d, 1 H, J=4.8 Hz, NCH₂C); 3.32 (d, 1H, J=4.8 Hz, NCH₂C); 3.21-3.08 (m, 2H, CH₂Argδ); 3.07 (d, 1 H, J=12.9 Hz, CCH₂Ph); 2.94 (s, 2H, CH₂Pbf); 2.93 (overload, 1 H, CCH₂Ph); 2.77-2.64 (m, 2H, CH_{β}Asp); 2.57 (s, 3H, CH₃Pbf); 2.51 (s, 3H, CH₃Pbf); 2.08 (s, 3H, CH₃Pbf); 1.90 (m, 2H, CH₂βArg); 1.59 (s, 3H, CH₃Aib); 1.49 (m, 2H, CH₂γArg); 1.45 (s, 6H, 2xCH₃Pbf); 1.40 (s, 9H, 3xCH₃); 1.39 (s, 3H, CH₃Aib); 0.89 (s, 3H, CH₃Aib). ¹³C-NMR (δ, ppm, CDCl₃): 173.5; 171.2; 171.1; 170.2; 166.7; 158.5; 156.4; 156.0; 138.3; 136.0; 135.6; 133.3; 133.0; 132.3; 130.4; 128.6; 128.5; 128.3; 128.2; 128.0; 127.7; 124.4; 117.2; 86.1; 82.0; 67.2; 66.7; 65.9; 59.9; 52.8; 51.6; 47.2; 43.1; 40.6; 38.4; 36.9; 28.6; 28.0; 25.7; 24.3; 23.1; 19.3; 17.8; 12.3. Anal. Calcd. For C₅₆H₇₁N₇O₁₂S: C, 63.08; H, 6.71; N, 9.20. Found: C, 63.10; H, 6.60; N, 9.10.

### EXAMPLE 22: Preparation of cyclo-[Asp(O^{t}Bu)-(beta-Lactam)-Arg(Pbf) (compound of formula (I'): R¹ = CH₂Ph; R² = R³ = methyl: R⁴ = H; R⁵ = Pbf; R⁶ = O^{t}Bu; 3S configuration).

A suspension of β-lactam peptide Cbz-Asp(O^{t}Bu)-(beta-Lactam)-Arg(Pbf)-OBn (95 mg, 0.17 mmol, prepared according to example 21) and Pd/C (38 mg, 10 %) in MeOH (10 mL) was stirred for 16h in a H₂ atmosphere generated by bubbling gas through the mixture. After reaction completion (checked by ¹H NMR analysis of aliquots) the mixture was filtered through celite. The solvent was evaporated "in vacuo", and the residual methanol was eliminated from the product by addition of dry CH₂Cl₂ (3 x 30 mL) and evaporation in the rotavapor. The intermediate N,C-deprotected pseudopeptide was immediately dissolved under N₂ atmosphere in dry DMF (200 mL) and the solution was cooled to -15°C. Anhydrous KHCO₃ (255 mg, 2.55 mmol), HOAT (37 mg, 0.27 mmol) and HATU (84 mg, 0.22 mmol) were added at -15°C, and the mixture was stirred for 20h at the same temperature. Evaporation of the solvent under vacuum provided a crude which was dissolved in EtOAc (20 mL), and the solution was washed consecutively with 1 M HCl (10 mL) and 5% aqueous NaHCO₃ (10 mL), dried and evaporated. The crude product was purified by preparative TLC (eluent: CH₂Cl₂/MeOH 9:1). Yield: 72 %. Oil.

[α]_{D}²⁵= -13.3 (c= 1.0, CH₂Cl₂); IR (cm⁻¹, KBr): 3439, 3332 (NH); 2933, 1747 (C=O), 1667(C=O), 1560, 1448 (NHC=O). HPLC-MS, MeOH/COOH m/z (Ion Source Type: ESI, negative polarity): 55.1, 57.1, 69.1, 71.1, 81.1, 83.1, 85.1, 95.1, 97.1, 109.1, 111.1, 123.1, 125.1. ¹H-NMR (δ, ppm, DMSOd⁶): 9.48 (s, 1 H, β-LactamNH); 8.21 (d, 1 H, J=9.9 Hz, NHαArg); 7.49 (d, 1 H, J=5.9 Hz, NHαAsp); 7.44-7.28 (m, 5H, Ar); 6.66-6.37 (bs., 2H, NHωAsp); 4.60 (m, 1 H, CHαAsp); 4.27 (m, 1 H, CHαArg); 3.43 (d, 1 H, J=5.0 Hz, NCH₂C ); 3.38 (d, 1 H, J=13.1 Hz, CCH₂Ph); 3.31 (d, 1 H, J=5.9Hz, NCH₂C); 3.08 (d, 1 H, J=13.1 Hz, CCH₂Ph); 2.96 (m, 2H, CH₂Argδ); 2.96 (s, 2H, CH₂Pbf); 2.77 (m, 1 H, CH_{β}Asp); 2.47 (s, 3H, CH₃Pbf); 2.42 (s, 3H, CH₃Pbf); 2.41 (overload, 1 H, CH_{β}Asp); 2.00 (s, 3H, CH₃Pbf); 1.70 (m, 1 H, CH₂βArg); 1.52 (m, 1 H, CH₂βArg); 1.41 (s, 6H, 2xCH₃Pbf); 1.35-1.33 (m, 2H, CH₂γArg); 1.34 (s, 9H, 3xCH₃); 0.80 (s, 3H, CH₃Aib); 0.79 (s, 3H, CH₃Aib). ¹³C-NMR (δ, ppm, CD₃OD): 172.5; 171.3; 168.9; 166.3; 157.0; 155.1; 136.6; 132.1; 130.6; 129.3; 126.7; 125.8; 123.1; 115.6; 84.8; 79.9; 65.0; 57.1; 52.0; 49.9; 41.2; 38.6; 36.1; 35.6; 35.1; 28.8; 25.9; 25.6; 24.5; 21.7; 20.9; 16.8; 15.6; 9.7. Anal. Calcd. For C₄₂H₆₁N₇O₉S(823,9978): C, 60.05; H, 7.32; N, 11.67. Found: C, 59.90; H, 6.90; N, 11.70. HRMS (m/z) 823.3938; C₄₃H₆₀N₈O₁₀S requires 823.3876.

### EXAMPLE 23: Preparation of cyclo-[Asp-(beta-Lactam)-Arg (compound of formula (I): R¹ = CH₂Ph; R²= R³= methyl; R⁴= H; 3S configuration), trifluoroacetate salt.

A solution of cyclo-[Asp(O^{t}Bu)-(beta-Lactam)-Arg(Pbf)] (48 mg, 0.24 mmol, prepared according to example 22) in CF₃CO₂H/CHCl₃ (3.6/0.4 mL) was stirred at 35°C for 31 h. Evaporation of the solution afforded a crude, which was poured into diisopropylether (30 mL) at 0°C and centrifugated. The solid was washed again with diisopropylether (2x10 mL) and dried under vacuum. Yield: 85%.

M.p: 240°C (dec.); [α]_{D}²⁵ = -85.6 (c= 0.5, MeOH); IR (cm⁻¹, KBr): 3462, (NH); 1742, 1644 (C=O); 1543 (NHCO). HPLC-MS, MeOH/COOH m/z (Ion Source Type: ESI, negative polarity): MS1=516.7(M*-CF₃COO⁻); MS2(516.8)= 443.2(100); 442.8(87); 225.1 (44); 369.2(37); 493.8(33); MS3(443.2)= 369.2(100); 385.7(61); 225.0(58); 516.2(56); 370.1(55)¹H-NMR (δ, ppm, D₂O/H₂O 1/9): 8.74 (s, 1 H, β-LactamNH); 8.49 (d, 1 H, J=9.8 Hz, NHαArg); 7.98 (d, 1 H, J=6.8 Hz, NHαAsp); 7.67-7.37 (m, 5H, Ar); 7.22 (bs., 2H, NHωAsp); 4.75 (bs., 1 H, CHαAsp); 4.43 (m, 1 H, CHαArg); 3.59 (d, 1 H, J=6.1 Hz, NCH₂C); 3.55 (d, 1 H, J=6.1 Hz, NCH₂C); 3.27 (d, 2H, J=13.2 Hz, CCH₂Ph); 3.17 (d, 2H, J=13.2 Hz, CCH₂Ph); 3.15 (m, 2H, HδArg); 2.96 (dd, 1 H, J₁=7.0, J₂=16.6 Hz, CH_{β}Asp); 2.82 (dd, 1 H, J₁=7.0, J₂=16.6 Hz, CH_{β}Asp); 1.88 (bs., 1 H, CHβArg); 1.72 (m, 1 H, CHβArg); 1.50 (m, 2H, CH₂γArg); 0.94 (s, 3H, CH₃Aib); 0.91 (s, 3H, CH₃Aib). ¹³C-NMR (δ, ppm, CD₃OD): 175.5; 173.9; 173.9; 171.8; 167.5; 158.4; 143.2; 135.0; 132.0; 129.2; 128.3; 127.2; 126.2; 68.1; 59.6; 59.7; 54.7; 51.6; 41.4; 38.0; 36.3; 31.1; 26.2; 24.3; 23.3; 15.0 Anal. Calcd. For C₂₆H₃₄F₃N₇O₈: C, 49.60; H, 5.44; N, 15.57. Found: C, 49.40; H, 5.80; N, 16.00.

### EXAMPLE 24: Endothelial cell adhesion assay

### Tested compounds

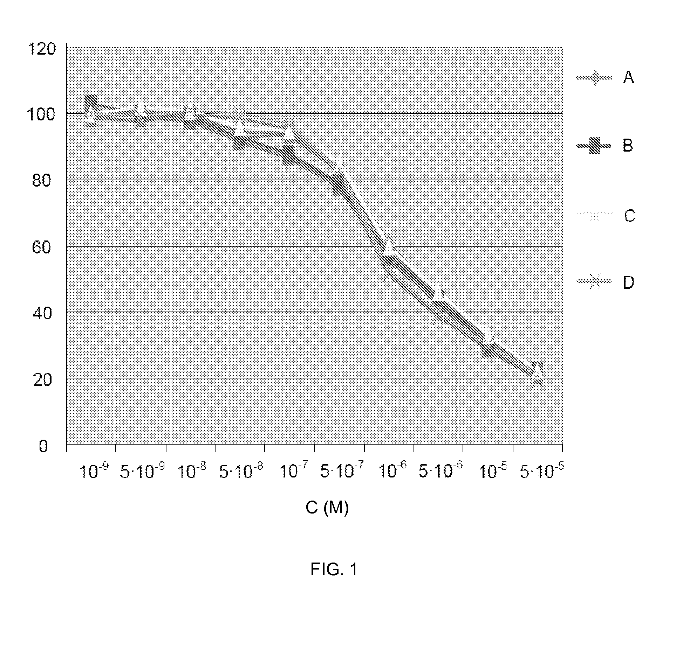

In this assay the compound of example 23 (B in FIG. 1), comparative cilengitide (C in FIG. 1) and two comparative RGD-cyclopentapeptides (A and D in FIG. 1) were assayed. The two assayed comparative RGD-cyclopentapeptides synthesized according to WO 2006/048473 have the following formula:

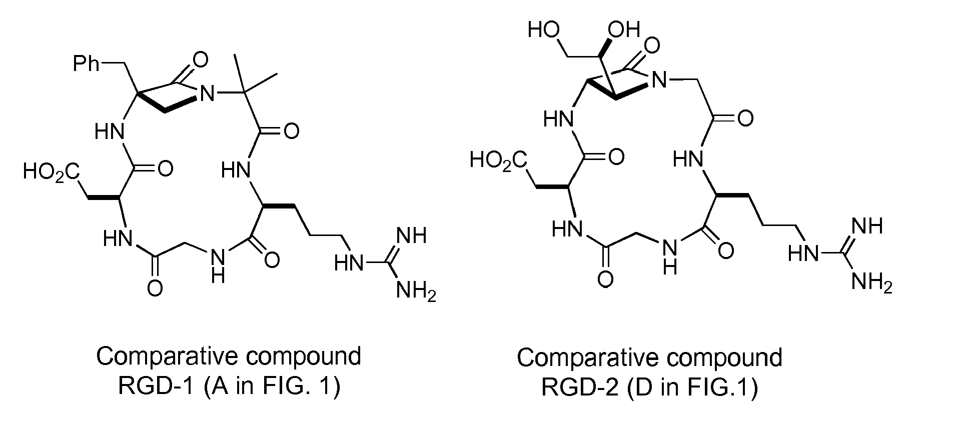

### 1.- Cell lines and reagents

Human umbilical endothelial cell (HUVEC) was purchased from Cambrex BioScience (USA). Cells were grown on the 0.5% gelatin coated plate in CS-C Complete Medium (Sigma) and were used for experiments after three passages. DLD-1, human colon cancer cell line was purchased from LGC/ATCC (Cat. Number CCL-221), and maintained in RPMI 1640 (Sigma) supplemented with 10% FBS (Sigma). Human vitronectin was from Sigma. Peptides to be assayed were dissolved in double-distilled water at a concentration of 10 mg/ml and stored in the dark at -80°.

### 2.- Endothelial cell adhesion assay

HUVEC cells were grown to subconfluent state and then harvested by 0.025% trypsin/EDTA (Sigma) in one minute. Cells were preincubated with various concentrations of peptides on ice for 15 min in CS-C Medium. Same volume of vehicle was added to cells as a control. Peptide-treated cells (5 x 10⁴ cells /100µl/well) were plated onto a 96-well microplate which was precoated with vitronectin (10 mg/ml in PBS), and incubated for 1 h at 37°C, 5% CO²/95% air to allow cell attachment. Cells were washed gently with PBS for three times to remove detached cells. Number of adherent cells was measured by Fluorescent Cell counting Kit (FCCK) at 535 nm (excitation at 485 nm) using a fluorescent plate reader (Bioscan). Experiments were done in triplicate wells and repeated four times.

### 3.- Statistics

Data are expressed as the means ± standard error of mean (SEM). Statistical differences among treatment groups were assessed by analysis of variance (ANOVA). The Tukey-Kramer test for post-hoc multiple comparisons were used when ANOVA was significant. Student's t test was used when appropriate. All statistical analyses were done using SPSS statistical software (Base version 12.0). A value of P <0.05 was considered significant.

FIG. 1 clearly shows that the compound of example 23 shows a comparable adhesion inhibition as cilengitide and the RGD-cyclopentapeptides. As it can be seen, peptide concentrations promote the occupancy of the receptor in an exponential manner. This occupancy avoids further adhesion with other putative interacting compound such as vitronectin.

### EXAMPLE 25: Gene regulation assay

### Tested compounds

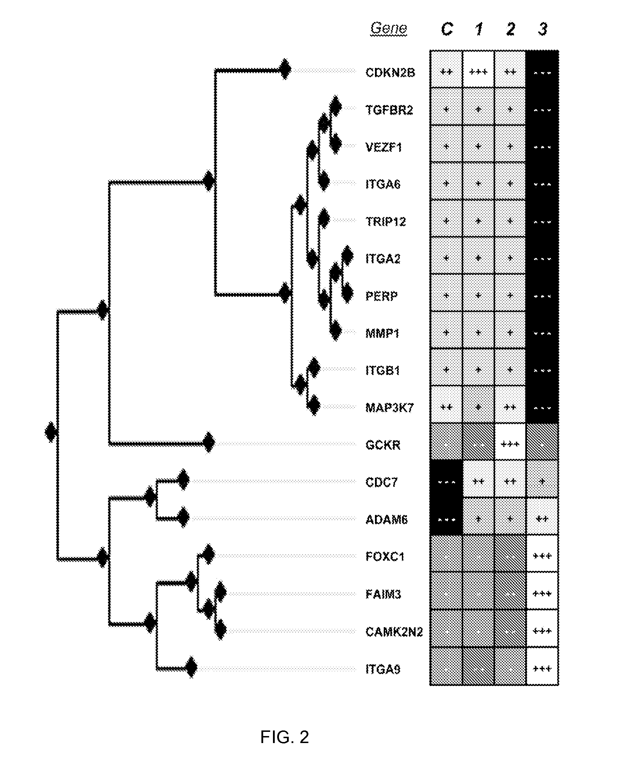

In this assay the compound of example 23 (3 in FIG. 2), comparative cilengitide (C in FIG. 2) and the above mentioned comparative RGD-cyclopentapeptides RGD-1 (2 in FIG. 2) and RGD-2 (1 in FIG. 2), were assayed.

### 1.- Cell culture and compound treatment

HUVEC cells were maintained in CS-C Complete Medium, grown at 50 per cent of confluency and then treated with 1.00E-05 M final concentration of the peptide compounds during 48 hours.

### 2.- RNA isolation and Gene-Expresion microarray assays

Total RNA was extracted from untreated control or RGD-compounds treated cells using TRIzol (Invitrogen) according to standard protocol. RNA was further purified with RNeasy Mini Kit (Qiagen) and the concentration and quality was assessed by NanoDrop spectrophotometer and Agilent 2100 Bioanalyzer, respectively. Only RNA Samples with a RIN number between 9-10 were used in the microarray assays.

### 3.3. Gene-Expresion microarray assay

In order to study the intracellular effect of all RGD-binding compounds, a cell-culture whole gene-expression assay was performed. HUVEC cells were treated separately with a 10⁻⁵M concentration of the test compounds during 48 hours. The simultaneous analysis of all human gene-expression by microarray let us identify which human gene(s) are induced, inhibited or unaffected after the test compounds were bound to the receptors of the cells.

Whole human gene expression microarray analysis was performed using Agilent Technologies (Santa Clara, CA). This platform comprises a two-color design with two RNA samples labeled with either Cy3 or Cy5 and hybridized to the same 44K Agilent microarray. Each array contains about 41,000 unique noncontrol 60-mer probes. RNAs from untreated and peptide treated-HUVEC cells were two-color labeled and hybridized to randomly chosen arrays a total of four times. A dye swap was performed for each sample on a different slide to remove any bias from the labeling dyes.

For each sample, 800 ng total RNA was reverse transcribed, linear amplified, and labeled with either Cy3 or Cy5 using Agilent's Low RNA Input Linear Amplification Kit PLUS, according to manufacturer's instructions. After labeling, samples were measured on a Nanodrop microarray module for labeling efficiency and quantification. Samples were then hybridized on Agilent 4 x 44K whole human genome GE arrays at 65°C for 17 h. After washing in GE washing buffer, the slide was scanned with Agilent Microarray Scanner. Feature extraction software (Version 9.5.3) was used to convert the image into gene expression data. Data were normalized by the Linear Lowess method. Genes that were 1.5-fold differentially expressed on 3 of 4 arrays were scored as significant. Furthermore, only genes with a p-value ≤ 0.05 based on a Student's t-test were selected. Mean fold change is mean of 4 arrays. Angiogenic-related genes were classified according to Gene Ontology.

The expression of a specific set of 17 angiogenic and adhesion-related genes are shown (FIG. 2).

| Official Symbol | Gene_ ID (*) | Gene Name |
|---|---|---|
| CDKN2B | 1030 | CDKN2B cyclin-dependent kinase inhibitor 2B (p15, inhibits CDK4) |
| TGFBR2 | 7048 | TGFBR2 transforming growth factor, beta receptor II (70/80kDa) |
| VEZF1 | 7716 | VEZF1 vascular endothelial zinc finger 1 |
| ITGA6 | 3655 | ITGA6 integrin, alpha 6 |
| TRIP12 | 9320 | TRIP12 thyroid hormone receptor interactor 12 |
| ITGA2 | 3673 | ITGA2 integrin, alpha 2 (CD49B, alpha 2 subunit of VLA-2 receptor) |
| PERP | 64065 | PERP PERP, TP53 apoptosis effector |
| MMP1 | 4312 | MMP1 matrix metallopeptidase 1 (interstitial collagenase) |
| ITGB1 | 3688 | ITGB1 integrin, beta 1 (fibronectin receptor, beta polypeptide, antigen CD29 includes MDF2, MSK12) |
| MAP3K7 | 6885 | MAP3K7 mitogen-activated protein kinase kinase kinase 7 |
| GCKR | 2646 | GCKR glucokinase (hexokinase 4) regulator |
| CDC7 | 8317 | CDC7 cell division cycle 7 homolog (S. cerevisiae) |
| ADAM6 | 8755 | ADAM6 ADAM metallopeptidase domain 6 (pseudogene) |
| FOXC1 | 2296 | FOXC1 forkhead box C1 |
| FAIM3 | 9214 | FAIM3 Fas apoptotic inhibitory molecule 3 |
| CAMK2N2 | 94032 | CAMK2N2 calcium/calmodulin-dependent protein kinase II inhibitor 2 |
| ITGA9 | 3680 | ITGA9 integrin, alpha 9 |

| | | |
|---|---|---|
| (*) http://www.ncbi.nlm.nih.gov/gene/ | | |

The symbols used in FIG. 2 are the following: (+++) mRNA concentration (fold-activation: >10); (++) mRNA concentration (fold-activation: 6-10); (+) mRNA concentration (fold-activation: 2-5); (---) mRNA concentration (fold-inhibition: >10); (--) mRNA concentration (fold- inhibition: 6-10); (-) mRNA concentration (fold- inhibition: 2-5).

10 of these genes were activated and 7 are blocked after treatment with the cilengitide (C). The pattern of gene-expression is mainly conserved (15/17 genes) in the compound 1, and 2 (14 of 17 genes). The lack of correlation in the expression of three genes (CDC7, ADAM6 and GCKR) shows the highly specific "in-vivo" cell activity after ligand-receptor binding. Surprisingly, the pattern of gene-expression after cell-treatment with the compound of example 23 (3 in FIG.2) was almost opposite (activation vs inhibition) in 15 of 17 genes if we compared with the cilengitide (C) (FIG. 2). These data suggest an "in-vivo" pro-angiogenic effect of the compounds of the invention, acting as an agonist ligand of the αᵥβ₃ integrin.

### REFERENCES CITED IN THE APPLICATION

Angew. Chem. Int. Ed. 1999, vol. 38, pp. 3056-3058
J. Org. Chem. 2001, vol. 66, pp. 6333-6338
J. Am. Chem. Soc. 2003, vol. 125, pp. 16243-16260
Org. Lett. 2007, vol. 9, pp. 101-104.
H. kessler et al, J. Biol. Chem. 1994, vol. 269, pp. 20233-20238
J. Org. Chem. 1997, vol. 62, pp. 2622-2624
Synlett 1996, vol.6, pp. 521-522

## Claims

1. A compound of formula (I), or a stereoisomer thereof, or a mixture of stereoisomers thereof, or a pharmaceutically acceptable salt thereof
cyclo[Arg-Asp-(beta-Lactam)] (I)
wherein (beta-Lactam) is a biradical of the formula (II)
wherein: 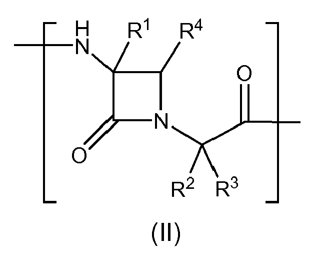 the terminal NH group of the (beta-Lactam) is attached to the α-carbonyl group of the aspartic residue (Asp), and the terminal carbonyl group of the (beta-Lactam) is attached to the α-amino group of the arginine residue (Arg);
R¹, R², R³ and R⁴ are radicals independently selected from the group consisting of hydrogen, (C₁-C₁₂)alkyl, (C₂-C₁₂)alkenyl, (C₂-C₁₂)alkynyl, (C₃-C₁₀)cycloalkyl, (C₂-C₉)heterocycloalkyl, (C₆-C₁₂)aryl, and (C₅-C₁₁)heteroaryl; wherein the radicals alkyl, alkenyl and alkynyl are optionally substituted with one or more substituents selected from the group consisting of halogen, -CN, -OR^{a}, ,-SR^{a} , -SOR^{a}, -SO₂R^{a}-, -NO₂, -N₃, -NR^{a}R^{b}, -COR^{a}, -CONR^{a}R^{b}, (C₆-C₁₂)aryl, and (C₅-C₁₁)heteroaryl; and the radicals cycloalkyl, heterocycloalkyl, aryl, and heteroaryl are optionally substituted with one or more substituents selected from the group consisting of halogen, (C₁-C₄)alkyl optionally substituted with one or more halogen atoms, -CN, -OR^{a}, -SR^{a}, -SOR^{a}, -SO₂R^{a}-, -NO₂, -N₃, -NR^{a}R^{b}, -COR^{a} and -CONR^{a}R^{b}, and 2,3,4,6-tetra-O-benzyl-α-D-mannosyl;
or alternatively, R² and R³ together with the carbon atom to which they are attached form a 3- to 7-membered monocyclic carbocyclic ring, partially unsaturated or saturated, optionally containing one or more heteroatoms selected from the group consisting of N, O and S, and being optionally substituted with one or more substituents selected from the group consisting of halogen, (C₁-C₄)alkyl optionally substituted with one or more halogen atoms, -CN, -OR^{a}, -SR^{a}, -SOR^{a}, -SO₂R^{a}-, -NO₂, -N₃, -NR^{a}R^{b}, -COR^{a} and -CONR^{a}R^{b}; and
R^{a} and R^{b} are independently H or (C₁-C₁₂)alkyl.

2. The compound according to claim 1, wherein R¹ is selected from the group consisting of hydrogen, (C₁-C₄)alkyl, (C₁-C₄)alkyl substituted with phenyl or with substituted phenyl, (C₁-C₄)alkyl substituted with triazolyl or substituted triazolyl, (C₁-C₄)alkyl substituted with -CHO, (C₂-C₄)alkenyl, and (C₂-C₄)alkynyl .

3. The compound according to any of the claims 1-2, wherein R¹ is selected from the group consisting of benzyl, benzyl substituted with one or more halogen atoms, and benzyl substituted with one or more halo(C₁-C₄)alkyl groups.

4. The compound according to any of the claims 1-3, wherein R² is selected from the group consisting of hydrogen, optionally substituted (C₁-C₄)alkyl, and optionally substituted phenyl.

5. The compound according to any of the claims 1-4, wherein R³ is hydrogen, or optionally substituted (C₁-C₄)alkyl.

6. The compound according to any of the claims 1-3, wherein R² and R³ form together an unsubstituted 3- to 5-membered saturated monocyclic carbocyclic ring.

7. The compound according to any of the claims 1-6, wherein R⁴ is hydrogen, or optionally substituted (C₂-C₉)heterocycloalkyl.

8. The compound according to claim 1, wherein:
R¹ = CH₂Ph; R² = R³ = R⁴ = H (3S configuration);
R¹ = CH₂Ph; R² = Ph, R³ = R⁴= H (3S, 1'R configuration);
R¹ = CH₂Ph; R² = Ph, R³ = R⁴= H (3S, 1'S configuration);
R¹ = CH₂Ph; R² = methyl, R³ = R⁴= H (3S, 1'S configuration);
R¹ = CH₂Ph; R² = methyl, R³ = R⁴= H (3S, 1'R configuration);
R¹ = 3-trifluoromethylbenzyl; R² = R³ = R⁴ = H (3S configuration);
R¹ = methyl; R² = R³ = R⁴ = H (3S configuration);
R¹ = methyl; R² = isopropyl; R³ = R⁴ = H (3R, (1'R configuration);
R¹ = CH₂Ph; R² = R³ = methyl; R⁴ = H (3S configuration);
R¹ = CH₂Ph; R² = R³ = together form a cyclopentyl ring; R⁴ = H (3S configuration);
R¹= CH₂C₆F₅; R² = R³ = methyl; R⁴ = H (3S configuration);
R¹= allyl; R² = R³ = methyl; R⁴ = H (3S configuration);
R¹= CH₂CHO; R² = R³ = methyl; R⁴ = H (3S configuration);
R¹= propargyl; R² = R³ = methyl; R⁴ = H (3S configuration);
R¹= [1-(2,3,4,6-tetra-O-benzyl-α-D-mannosyl)-1,2,3-triazol-4-yl]methyl; R² = R³ = methyl; R⁴ = H (3S configuration);
R¹ = R² = R³ = H; R⁴ = (4S)-2,2-dimethyl-1,3-dioxolan-4-yl (3S 4R configuration); or
R¹ = R² = R³ = H; R⁴ = (4S)-2,2-dimethyl-1,3-dioxolan-4-yl (3R 4R configuration).

9. A process for preparing a compound of formula (I) as defined in any of the claims 1-8, which comprises:
a) reacting a compound of formula H-Z-OH (III) with a condensing agent, optionally in the presence of a base, wherein Z is a biradical selected from the group consisting of -Arg(R⁵)-Asp(R⁶)-(beta-Lactam)-, -Asp(R⁶)-(beta-Lactam)-Arg(R⁵)-, and -(beta-Lactam)-Arg(R⁵)-Asp(R⁶)-; R⁵ is H or an amino protecting group PG¹; R⁶ is H or a carboxyl protecting group PG²; and (beta-Lactam) is a biradical of the formula (II) as defined in any of the claims 1-8; and
b) if necessary, removing the protecting groups PG¹ and PG²; and
c) optionally converting a basic or acidic compound of the formula (I) obtained in step a) or b) into one of its salts by treatment with an acid or base.

10. A compound of formula (III)
H-Z-OH (III)
wherein
Z is a biradical selected from the group consisting of
- Arg(R⁵)-Asp(R⁶)-(beta-Lactam)-, -Asp(R⁶)-(beta-Lactam)-Arg(R⁵)-, and
- (beta-Lactam)-Arg(R⁵)-Asp(R⁶)-;
R⁵ is H or an amino protecting group PG¹;
R⁶ is H or a carboxyl protecting group PG²; and
(beta-Lactam) is a biradical of the formula (II) as defined in any of the claims 1-7.

11. A compound of formula (I')
cyclo[Arg(R⁵)-Asp(R⁶)-(beta-Lactam)] (I')
wherein
R⁵ is H or an amino protecting group PG¹;
R⁶ is H or a carboxyl protecting group PG²; and
(beta-Lactam) is a biradical of the formula (II) as defined in any of the claims 1-7.

12. A pharmaceutical composition which comprises an effective amount of a compound of formula (I) as defined in any of the claims 1-8, together with one or more pharmaceutically acceptable excipients or carriers.

13. A compound of formula (I) as defined in any of the claims 1-8 for use in medicine.

14. The compound of formula (I) for use according to claim 12, for the treatment of diseases mediated by αᵥβ₃ integrin agonists.

15. The compound of formula (I) for use according to claim 12, for the stimulation of angiogenesis to treat diabetic ulcers, blood vessels wounds on heart or human melanoma.
